(19) Europäisches Patentamt
European Patent Office
Office européen des brevets

(11) Veröffentlichungsnummer: **0 238 021**
**A1**

(12) **EUROPÄISCHE PATENTANMELDUNG**

(21) Anmeldenummer: **87103795.8**

(22) Anmeldetag: **16.03.87**

(51) Int. Cl.4: **C07D 261/08**, A01N 43/80

(30) Priorität: **19.03.86 DE 3609181**

(43) Veröffentlichungstag der Anmeldung:
**23.09.87 Patentblatt 87/39**

(84) Benannte Vertragsstaaten:
**BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Kolassa, Dieter, Dr.**
**Moltkestrasse 8**
**D-6700 Ludwigshafen(DE)**
Erfinder: **Keil, Michael, Dr.**
**Fontanestrasse 4**
**D-6713 Freinsheim(DE)**
Erfinder: **Schirmer, Ulrich, Dr.**
**Berghalde 79**
**D-6900 Heidelberg(DE)**
Erfinder: **Theobald, Hans, Dr.**
**Queichstrasse 6**
**D-6703 Limburgerhof(DE)**
Erfinder: **Becker Rainer, Dr.**
**Im Haseneck 22**
**D-6702 Bad Duerkheim(DE)**
Erfinder: **Wuerzer, Bruno, Dr.**
**Ruedigerstrasse 13**
**D-6701 Otterstadt(DE)**
Erfinder: **Meyer, Norbert, Dr.**
**Dossenheimer Weg 22**
**D-6802 Ladenburg(DE)**

(54) **Cyclohexenonderivate, Verfahren zu ihrer Herstellung und ihre Verwendung zur Bekämpfung unerwüschten Pflanzenwachstums.**

(57) Cyclohexenonderivate der Formel

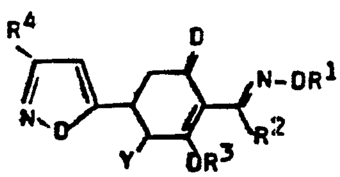

(I),

in der
$R^1$ Alkyl, Alkenyl, Alkinyl, Halogenalkyl und Halogenalkenyl, Alkoxyalkyl, Cycloalkylmethyl, gegebenenfalls substituiert, Benzyl, gegebenenfalls substituiert, einen 5-Ring-Heterocyclus, der über eine Methyleneinheit mit dem Sauerstoff verbunden ist,
$R^2$ Alkyl,
$R^3$ Wasserstoff, Alkylcarbonyl, Alkenylcarbonyl, Benzoyl, gegebenenfalls substituiert, Trialkylsilyl, Alkyl-und Arylsulfonyl Dialkylphosphono und -thiophosphono, organisches oder anorganisches Kation,
$R^4$ Alkyl, Alkenyl oder Alkinyl, gegebenenfalls substituiert, Cycloalkyl, gegebenenfalls substituiert,

EP 0 238 021 A1

Xerox Copy Centre

Y Wasserstoff, Methoxycarbonyl, Cyan, Alkyl und Halogen bedeuten und diese enthaltende Herbizide.

# Cyclohexenonderivate, Verfahren zu ihrer Herstellung und ihre Verwendung zur Bekämpfung unerwünschten Pflanzenwachstums

Die vorliegende Erfindung betrifft Cyclohexenonderivate, Verfahren zu ihrer Herstellung sowie Herbizide, die diese Verbindungen als Wirkstoffe enthalten.

Die herbizide Wirkung von 3-Hydroxy-2-cyclohexen-1-on-oximetherderivaten, die in 5-Position einen fünfgliedrigen heterocyclischen Rest tragen, ist aus EP 162 224 und EP 125 094 bekannt.

Es wurde gefunden, daß Cyclohexenonderivate der Formel

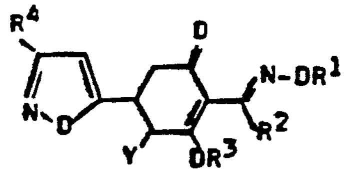

(I),

in der

$R^1$Alkyl mit 1 bis 4 Kohlenstoffatomen, Alkenyl mit 3 bis 4 Kohlenstoffatomen, Alkinyl mit 3 bis 4 Kohlenstoffatomen, Halogenalkyl und Halogenalkenyl mit 2 bis 4 Kohlenstoffatomen und 1 bis 3 Halogenatomen, Alkoxyalkyl mit 2 bis 4 Kohlenstoffatomen, Cycloalkylmethyl mit 4 bis 7 Kohlenstoffatomen, gegebenenfalls durch $C_1$-$C_4$-Alkyl oder Halogen substituiert, Benzyl, gegebenenfalls durch $C_1$-$C_4$-Alkyl, $C_1$-$C_4$-Alkoxy, $C_1$-$C_4$-Alkylthio, Halogen, Cyan, Trifluormethyl oder Nitro substituiert, einen 5-Ring-heterocyclus, der über eine Methyleneinheit mit dem Sauerstoff verbunden ist und 1 oder 2 Heteroatome aus der Gruppe N, O, S und bis zu 2 Doppelbindungen und bis zu 2 Substituenten aus der Gruppe $C_1$-$C_4$-Alkyl, $C_1$-$C_4$-Alkoxy oder Halogen enthalten kann,

$R^2$ Alkyl mit 1 bis 4 Kohlenstoffatomen,

$R^3$ Wasserstoff, Alkylcarbonyl mit 1 bis 20 Kohlenstoffkette, Alkenylcarbonyl mit 1 bis 20 Kohlenstoffatomen und einer Doppelbindung, Benzoyl, gegebenenfalls durch $C_1$-$C_8$-Alkyl substituiert, Trialkylsilyl mit 1 bis 4 Kohlenstoffatomen je Alkylrest, Alkyl-und Arylsulfonyl mit 1 bis 7 Kohlenstoffatomen, Dialkylphosphono und -thiophosphono mit 1 bis 4 Kohlenstoffatomen je Alkylrest, ein organisches oder anorganisches Kation,

$R^4$ $C_2$-$C_{20}$-Alkyl, Alkenyl oder Alkinyl, wobei die Kohlenstoffatomen bis zu zwei Doppel-bzw. Dreifachbindungen enthalten und durch $C_1$-$C_4$-Alkyl, $C_1$-$C_4$-Alkoxy oder $C_1$-$C_4$-Alkylthio substituiert sein kann, $C_3$-$C_6$-Cycloalkyl, gegebenenfalls durch $C_1$-$C_4$-Alkyl, $C_1$-$C_4$-Alkoxy oder

$C_1$-$C_4$-Alkylthio substituiert, $C_3$-$C_8$-Alkyl mit 2 O oder S-Atomen in der Alkylkette, Methyl substituiert durch $C_1$-$C_4$-Alkoxy oder $C_1$-$C_4$-Alkylthio,

Y Wasserstoff, Methoxycarbonyl, Cyan, Alkyl und Halogen bedeutet,

eine gute herbizide Wirkung vorzugsweise gegen Arten aus der Familie der Gräser (Gramineen) besitzen. Sie sind verträglich und somit selektiv in breitblättrigen Kulturen sowie in monokotylen Gewächsen, welche nicht zu den Gramineen zählen. Einige Verbindungen verhalten sich auch in Gramineenkulturen, wie z.B. Weizen, selektiv und bekämpfen gleichzeitig unerwünschte Gräser.

Beispiele für den Rest $R^1$ in Formel I sind Methyl, Ethyl, n-Propyl, Allyl, (E)-2-Butenyl, 2-Fluorethyl, 2-Chlorethyl, (E)-3-Chlor-2-propenyl, 2,3,3-Trichlor-2-propenyl, Propargyl, Methoxymethyl, Methoxyethyl, 3,3-Dichlorcyclopropylmethyl, 3-Chlorbenzyl, 4-Cyanbenzyl, 3-Methyl-5-isoxazolylmethyl, 5-Chlor-2-thenyl, 5-Brom-2-thenyl, Thenyl = Methylenthienyl.

$R^2$ in Formel I steht für unverzweigte und verzweigte Alkylreste mit 1 bis 4 Kohlenstoffatomen, d.h. für Methyl, Ethyl, n-Propyl, i-Propyl, n-Butyl, s-Butyl, i-Butyl und t-Butyl.

Beispiele für $R^3$ sind Wasserstoff, Acetyl, Propionyl, Butyryl, i-Butyryl, Pivaloyl, Valeroyl, Caproyl, Caprinoyl, Lauroyl, Palmitoyl, Stearoyl, Oleoyl, Benzoyl, 4-Hexylbenzoyl, Methylsulfonyl, p-Tolylsulfonyl, Trimethylsilyl, Diethylphosphono, Diethylthiophosphono, weiterhin Alkalimetallkationen, insbesondere Natrium und Kalium, Erdalkalimetallkationen, insbesondere Calcium, Magnesium und Barium, Mangan-, Kupfer-, Zink-oder Eisenkationen sowie Ammonium-, Phosphonium-, Sulfonium-und Sulfoxoniumkationen wie Ammonium, Tetraalkylammonium, Benzyltrialkylammonium, Trialkylsulfonium oder Trialkylsulfoxonium.

Beispiele für $R^4$ sind z.B. $C_2$-$C_{10}$-Alkyl, Ethyl, n-Propyl, i-Propyl, n-Butyl, s-Butyl, t-Butyl, i-Butyl, 2,4,4-Trimethylpentyl, 1-Ethylpentyl, 2,6-Dimethylheptyl, Cyclopropyl, Cyclohexyl, Methoxymethyl, Ethoxymethyl, Methylthiomethyl, Ethylthiomethyl, 1-Methoxyethyl, 2-Methylthioethyl, 1,3-Dimethoxypropyl, 2-Ethoxyethyl, 1-Methyl-2-methylthioethyl, 2-Methyl-1-methylthiomethyl-propyl, 1-Methylthiomethyl-butyl, 2-Methyl-1-propenyl.

Beispiele für Y sind: Wasserstoff, Methoxycarbonyl, Cyan, Methyl, Chlor. Die Cyclohexenonderivate der Formel I können durch Umsetzung von Verbindungen der Formel II

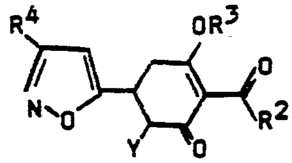

(II),

in der $R^2$, $R^3$, $R^4$ und Y die obengenannten Bedeutungen haben, mit Alkoxyammoniumverbindungen der Formel $R^1ONH_2$ . HX, in der X eine Anion bedeutet, erhalten werden. Hierzu setzt man die beiden Reaktionsteilnehmer in einem Lösungsmittel in Gegenwart einer Base bei einer ausreichenden Temperatur, die zwischen 0°C und dem Siedepunkt des Lösungsmittels liegen kann, um. Geeignete Basen sind beispielsweise Carbonate, Hydrogencarbonate, Acetate, Alkoholate, Oxide bzw. Hydroxide von Alkali-oder Erdalkalimetallen, insbesondere von Natrium, Kalium, Magnesium, Calcium oder organischen Basen, wie Pyridin oder tertiäre Amine.

Als Lösungsmittel sind beispielsweise Dimethylsulfoxid, Alkohole wie Methanol, Ethanol, Isopropanol, aromatische Kohlenwasserstoffe wie Benzol, Toluol, chlorierte Kohlenwasserstoffe wie Chloroform, Dichlorethan, aliphatische Kohlenwasserstoffe wie Hexan, Cyclohexan, Ester wie Essigsäureethylester, Ether wie Dioxan, Tetrahydrofuran geeignet.

Die Umsetzung ist nach wenigen Stunden beendet, das Reaktionsprodukt kann durch Einengen der Mischung, Verteilung des Rückstandes in Methylenchlorid/Wasser und Abdestillieren des Lösungsmittels unter vermindertem Druck isoliert werden.

Die Verbindungen der Formel I können außerdem durch Umsetzen der Verbindungen II mit entsprechenden Alkoxyaminen der Formel $R^1ONH_2$ in einem geeigneten Verdünnungsmittel erhalten werden, eine geeignete Reaktionstemperatur liegt z.B. zwischen 15 und 70°C. Das Alkoxyamin kann in Form einer wäßrigen Lösung eingesetzt werden; je nach dem verwendeten Lösungsmittel für den anderen Reaktionsteilnehmer erhält man ein ein-oder zweiphasiges Reaktionsgemisch.

Geeignete Lösungsmittel für diese Umsetzung sind beispielsweise Alkohole wie Methanol, Ethanol, Isopropanol, Cyclohexanol, aliphatische und aromatische, gegebenenfalls chlorierte Kohlenwasserstoffe, wie Hexan, Cyclohexan, Methylenchlorid, Toluol, Dichlorethan, Ester wie Essigsäureethylester, Nitril wie Acetonitril, cyclische Ether wie Tetrahydrofuran.

Alkalimetallsalze der Verbindungen I können durch Behandeln der 3-Hydroxyverbindungen mit Natrium-oder Kaliumhydroxid oder -alkoholat in wäßriger Lösung oder in einem organischen Lösungsmittel wie Methanol, Ethanol, Aceton, Toluol erhalten werden.

Andere Metallsalze, z.B. die Mangan-, Kupfer-, Zink-, Eisen-, Calcium-, Magnesium-und Bariumsalze können aus den Natriumsalzen in üblicher Weise hergestellt werden, ebenso Ammonium-und Phosphoniumsalze mittels Ammoniak, Phosphonium-, Sulfonium-oder Sulfoxoniumhydroxiden.

Die Verbindungen I können z.B. aus den entsprechenden Cyclohexan-1,3-dionen der Formel III

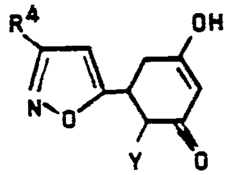

(III),

nach bekannten Methoden [Tetrahedron Lett., 2491 (1975)] hergestellt werden.

Es ist auch möglich, Verbindungen der Formel I über die Zwischenstufe der Enolester, die bei der Umsetzung von Verbindungen der Formel III mit Säurechloriden in Gegenwart von Basen anfallen und anschließend mit bestimmten Imidazol-oder Pyridinderivaten umgelagert werden, herzustellen (JP-OS 79/063052).

Zu den Verbindungen der Formel I gelangt man nach bekannten Verfahren, wie dies aus folgendem Schema hervorgeht:

Z steht dabei für einen 5-Isoxazolylrest, der in 3-Position durch $R^4$ substituiert ist.

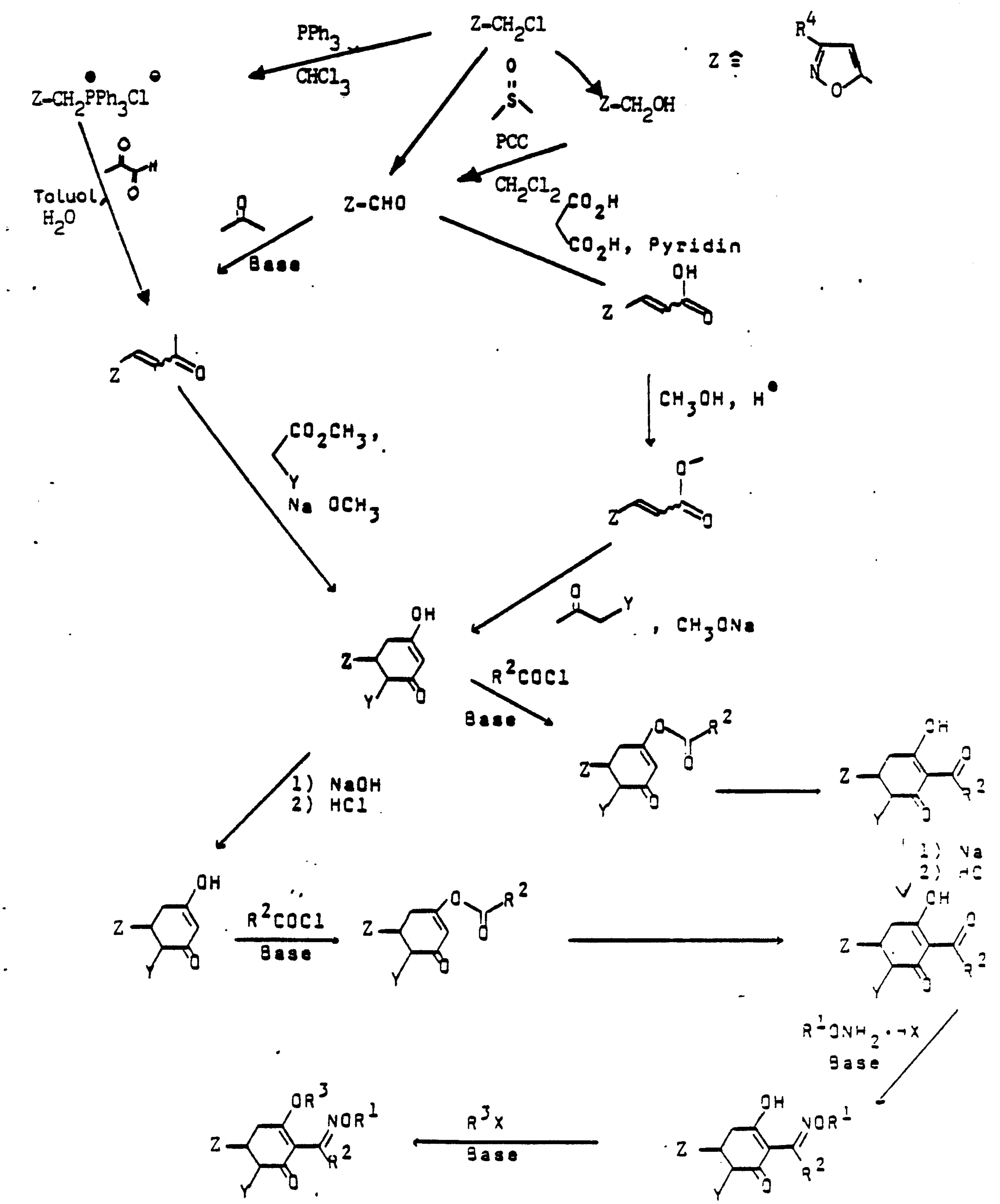
PPh$_3$
CHCl$_3$
PCC
CH$_2$Cl$_2$
Pyridin
Toluol
H$_2$O
Base
CH$_3$OH, H$^⊕$
Na OCH$_3$
CH$_3$ONa
R$^2$COCl
Base
1) NaOH
2) HCl
R$^2$COCl
Base
1) Na
2) HC
R$^1$ONH$_2$·HX
Base
R$^3$X
Base

Als Ausgangsmaterialien eignen sich die 5-Chlormethylisoxazole Z-$CH_2$-Cl, die durch 1,3-dipolare Cycloaddition von Propargylchlorid an Hydroxamsäurechloride oder Nitriloxide zugänglich sind. Die dabei üblichen Methoden sind bekannt.

In gleicher Weise geeignet sind auch die Verbindungen, die statt des Chlors ein anderes Halogenatom enthalten.

Über die Zwischenstufe der 5-Hydroxymethylverbindung oder auf direktem Weg gelangt man von dem Chlormethylisoxazol Z-$CH_2$-Cl nach bekannten Verfahren zu den 5-Formylisoxazolen Z-CHO, die sich ebenfalls als Ausgangsmaterialien anbieten.

Das folgende Beispiel erläutert die Herstellung der neuen Verbindungen. Dabei verhalten sich Gewichtsteile zu Volumenteilen wie Kilogramm zu Liter.

Beispiel

Eine Mischung aus 30.0 g (0.11 mol) 3-Hydroxy-5-(3-isopropylisoxazolyl)-2-propionyl-2-cyclohexen-1-on, 10.1 g (0.12 mol) Natriumhydrogencarbonat, 11.7 g (0.12 mol) Ethoxyaminhydrochlorid in 350 ml Ethanol wird 1 h bei Raumtemperatur gerührt. Dann wird das Lösungsmittel abgetrennt, der Rückstand in 300 ml Eiswasser verrührt, abgesaugt, mit Wasser gewaschen und im Vakuum getrocknet. Ausbeute: 29 g - (82 % der Theorie) eines beigefarbenen Feststoffs. Fp: 84-85°C (= Wirkstoff 29).

In entsprechende Weise können die in der Tabelle aufgeführten Verbindungen der Formel I hergestellt werden, die durch ihren Schmelzpunkt (Schmp.) charakterisiert werden. In Tabelle 2 sind deren $^1$H-NMR-Daten und Elementaranalysen niedergelegt. Verbindungen, bei denen die physikalischen Daten fehlen, können nach ähnlichen Verfahren erhalten werden.

Die $^1$H-NMR-Spektren wurden in Deuteriochloroform oder Hexadeuteriosulfoxid als Lösungsmittel mit Tetramethylsilan als innerem Standard aufgenommen. Die chemischen Verschiebungen werden in (ppm) registriert. die Multiplizitäten werden wie folgt angegeben: s = Singulett, d = Dublett, t = Triplett, q = Quartett, m = Multiplett.

<u>Tabelle 1</u> - Wirkstoffliste

| | $R^4$ | $R^3$ | $R^2$ | $R^1$ | Schm./°C |
|---|---|---|---|---|---|
| 1 | Ethyl | H | Methyl | Ethyl | |
| 2 | Ethyl | H | Methyl | Allyl | |
| 3 | Ethyl | H | Methyl | (E)-3-Chlor-2-propenyl | |
| 4 | Ethyl | H | Methyl | (E)-2-Butenyl | |
| 5 | Ethyl | H | Ethyl | Ethyl | 79- 81 |
| 6 | Ethyl | H | Ethyl | Allyl | 61- 63 |
| 7 | Ethyl | H | Ethyl | (E)-3-Chlor-2-propenyl | 86- 88 |
| 8 | Ethyl | H | Ethyl | (E)-2-Butenyl | 80- 82 |
| 9 | Ethyl | H | n-Propyl | Ethyl | |
| 10 | Ethyl | H | n-Propyl | Allyl | |
| 11 | Ethyl | H | n-Propyl | (E)-3-Chlor-2-propenyl | |
| 12 | Ethyl | H | n-Propyl | (E)-2-Butenyl | |
| 13 | n-Propyl | H | Methyl | Ethyl | |
| 14 | n-Propyl | H | Methyl | Allyl | |
| 15 | n-Propyl | H | Methyl | (E)-3-Chlor-2-propenyl | |
| 16 | n-Propyl | H | Methyl | (E)-2-Butenyl | |
| 17 | n-Propyl | H | Ethyl | Ethyl | |
| 18 | n-Propyl | H | Ethyl | Allyl | |
| 19 | n-Propyl | H | Ethyl | (E)-3-Chlor-2-propenyl | |
| 20 | n-Propyl | H | Ethyl | (E)-2-Butenyl | |
| 21 | n-Propyl | H | n-Propyl | Ethyl | |
| 22 | n-Propyl | H | n-Propyl | Allyl | |
| 23 | n-Propyl | H | n-Propyl | (E)-3-Chlor-2-propenyl | |
| 24 | n-Propyl | H | n-Propyl | (E)-2-Butenyl | |
| 25 | i-Propyl | H | Methyl | Ethyl | |
| 26 | i-Propyl | H | Methyl | Allyl | 78- 80 |

| | $R^4$ | $R^3$ | $R^2$ | $R^1$ | Schm./°C |
|---|---|---|---|---|---|
| 27 | i-Propyl | H | Methyl | (E)-3-Chlor-2-propenyl | |
| 28 | i-Propyl | H | Methyl | (E)-2-Butenyl | |
| 29 | i-Propyl | H | Ethyl | Ethyl | 84- 85 |
| 30 | i-Propyl | H | Ethyl | Allyl | 71- 74 |
| 31 | i-Propyl | H | Ethyl | (E)-3-Chlor-2-propenyl | 103-106 |
| 32 | i-Propyl | H | Ethyl | (E)-2-Butenyl | 107-110 |
| 33 | i-Propyl | H | n-Propyl | Ethyl | 60- 61 |
| 34 | i-Propyl | H | n-Propyl | Allyl | |
| 35 | i-Propyl | H | n-Propyl | (E)-3-Chlor-2-propenyl | |
| 36 | i-Propyl | H | n-Propyl | (E)-2-Butenyl | 80-84 |
| 37 | n-Butyl | H | Methyl | Ethyl | |
| 38 | n-Butyl | H | Methyl | Allyl | |
| 39 | n-Butyl | H | Methyl | (E)-3-Chlor-2-propenyl | |
| 40 | n-Butyl | H | Methyl | (E)-2-Butenyl | |
| 41 | n-Butyl | H | Ethyl | Ethyl | |
| 42 | n-Butyl | H | Ethyl | Allyl | |
| 43 | n-Butyl | H | Ethyl | (E)-3-Chlor-2-propenyl | |
| 44 | n-Butyl | H | Ethyl | (E)-2-Butenyl | |
| 45 | n-Butyl | H | n-Propyl | Ethyl | |
| 46 | n-Butyl | H | n-Propyl | Allyl | |
| 47 | n-Butyl | H | n-Propyl | (E)-3-Chlor-2-propenyl | |
| 48 | n-Butyl | H | n-Propyl | (E)-2-Butenyl | |
| 49 | s-Butyl | H | Methyl | Ethyl | |
| 50 | s-Butyl | H | Methyl | Allyl | |
| 51 | s-Butyl | H | Methyl | (E)-3-Chlor-2-propenyl | |
| 52 | s-Butyl | H | Methyl | (E)-2-Butenyl | |
| 53 | s-Butyl | H | Ethyl | Ethyl | |
| 54 | s-Butyl | H | Ethyl | Allyl | |

| | $R^4$ | $R^3$ | $R^2$ | $R^1$ | Schm./°C |
|---|---|---|---|---|---|
| 55 | s-Butyl | H | Ethyl | (E)-3-Chlor-2-propenyl | |
| 56 | s-Butyl | H | Ethyl | (E)-2-Butenyl | |
| 57 | s-Butyl | H | n-Propyl | Ethyl | |
| 58 | s-Butyl | H | n-Propyl | Allyl | |
| 59 | s-Butyl | H | n-Propyl | (E)-3-Chlor-2-propenyl | |
| 60 | s-Butyl | H | n-Propyl | (E)-2-Butenyl | |
| 61 | t-Butyl | H | Methyl | Ethyl | |
| 62 | t-Butyl | H | Methyl | Allyl | |
| 63 | t-Butyl | H | Methyl | (E)-3-Chlor-2-propenyl | |
| 64 | t-Butyl | H | Methyl | (E)-2-Butenyl | |
| 65 | t-Butyl | H | Ethyl | Ethyl | 94- 97 |
| 66 | t-Butyl | H | Ethyl | Allyl | 92- 94 |
| 67 | t-Butyl | H | Ethyl | (E)-3-Chlor-2-propenyl | 120-122 |
| 68 | t-Butyl | H | Ethyl | (E)-2-Butenyl | 110-113 |
| 69 | t-Butyl | H | n-Propyl | Ethyl | 75- 78 |
| 70 | t-Butyl | H | n-Propyl | Allyl | 83- 85 |
| 71 | t-Butyl | H | n-Propyl | (E)-3-Chlor-2-propenyl | 128-131 |
| 72 | t-Butyl | H | n-Propyl | (E)-2-Butenyl | 118-120 |
| 73 | i-Butyl | H | Methyl | Ethyl | |
| 74 | i-Butyl | H | Methyl | Allyl | |
| 75 | i-Butyl | H | Methyl | (E)-3-Chlor-2-propenyl | |
| 76 | i-Butyl | H | Methyl | (E)-2-Butenyl | |
| 77 | i-Butyl | H | Ethyl | Ethyl | |
| 78 | i-Butyl | H | Ethyl | Allyl | |
| 79 | i-Butyl | H | Ethyl | (E)-3-Chlor-2-propenyl | |
| 80 | i-Butyl | H | Ethyl | (E)-2-Butenyl | |
| 81 | i-Butyl | H | n-Propyl | Ethyl | |
| 82 | i-Butyl | H | n-Propyl | Allyl | |

| | $R^4$ | $R^3$ | $R^2$ | $R^1$ | Schm./°C |
|---|---|---|---|---|---|
| 83 | 1-Butyl | H | n-Propyl | (E)-3-Chlor-2-propenyl | |
| 84 | 1-Butyl | H | n-Propyl | (E)-2-Butenyl | |
| 85 | 2,4,4-Trimethyl-pentyl | H | Methyl | Ethyl | |
| 86 | 2,4,4-Trimethyl-pentyl | H | Methyl | Allyl | |
| 87 | 2,4,4-Trimethyl-pentyl | H | Methyl | (E)-3-Chlor-2-propenyl | |
| 88 | 2,4,4-Trimethyl-pentyl | H | Methyl | (E)-2-Butenyl | |
| 89 | 2,4,4-Trimethyl-pentyl | H | Ethyl | Ethyl | |
| 90 | 2,4,4-Trimethyl-pentyl | H | Ethyl | Allyl | |
| 91 | 2,4,4-Trimethyl-pentyl | H | Ethyl | (E)-3-Chlor-2-propenyl | |
| 92 | 2,4,4-Trimethyl-pentyl | H | Ethyl | (E)-2-Butenyl | |
| 93 | 2,4,4-Trimethyl-pentyl | H | n-Propyl | Ethyl | |
| 94 | 2,4,4-Trimethyl-pentyl | H | n-Propyl | Allyl | |
| 95 | 2,4,4-Trimethyl-pentyl | H | n-Propyl | (E)-3-Chlor-2-propenyl | |
| 96 | 2,4,4-Trimethyl-pentyl | H | n-Propyl | (E)-2-Butenyl | |
| 97 | 1-Ethylpentyl | H | Methyl | Ethyl | |
| 98 | 1-Ethylpentyl | H | Methyl | Allyl | |
| 99 | 1-Ethylpentyl | H | Methyl | (E)-3-Chlor-2-propenyl | |

| | $R^4$ | $R^3$ | $R^2$ | $R^1$ | Schm./°C |
|---|---|---|---|---|---|
| 100 | 1-Ethylpentyl | H | Methyl | (E)-2-Butenyl | |
| 101 | 1-Ethylpentyl | H | Ethyl | Ethyl | |
| 102 | 1-Ethylpentyl | H | Ethyl | Allyl | |
| 103 | 1-Ethylpentyl | H | Ethyl | (E)-3-Chlor-2-propenyl | |
| 104 | 1-Ethylpentyl | H | Ethyl | (E)-2-Butenyl | |
| 105 | 1-Ethylpentyl | H | n-Propyl | Ethyl | |
| 106 | 1-Ethylpentyl | H | n-Propyl | Allyl | |
| 107 | 1-Ethylpentyl | H | n-Propyl | (E)-3-Chlor-2-propenyl | |
| 108 | 1-Ethylpentyl | H | n-Propyl | (E)-2-Butenyl | |
| 109 | 2,6-Dimethylheptyl | H | Methyl | Ethyl | |
| 110 | 2,6-Dimethylheptyl | H | Methyl | Allyl | |
| 111 | 2,6-Dimethylheptyl | H | Methyl | (E)-3-Chlor-2-propenyl | |
| 112 | 2,6-Dimethylheptyl | H | Methyl | (E)-2-Butenyl | |
| 113 | 2,6-Dimethylheptyl | H | Ethyl | Ethyl | |
| 114 | 2,6-Dimethylheptyl | H | Ethyl | Allyl | |
| 115 | 2,6-Dimethylheptyl | H | Ethyl | (E)-3-Chlor-2-propenyl | |
| 116 | 2,6-Dimethylheptyl | H | Ethyl | (E)-2-Butenyl | |
| 117 | 2,6-Dimethylheptyl | H | n-Propyl | Ethyl | |
| 118 | 2,6-Dimethylheptyl | H | n-Propyl | Allyl | |
| 119 | 2,6-Dimethylheptyl | H | n-Propyl | (E)-3-Chlor-2-propenyl | |
| 120 | 2,6-Dimethylheptyl | H | n-Propyl | (E)-2-Butenyl | |
| 121 | Cyclopropyl | H | Methyl | Ethyl | |
| 122 | Cyclopropyl | H | Methyl | Allyl | |
| 123 | Cyclopropyl | H | Methyl | (E)-3-Chlor-2-propenyl | |
| 124 | Cyclopropyl | H | Methyl | (E)-2-Butenyl | |
| 125 | Cyclopropyl | H | Ethyl | Ethyl | |
| 126 | Cyclopropyl | H | Ethyl | Allyl | |
| 127 | Cyclopropyl | H | Ethyl | (E)-3-Chlor-2-propenyl | |

| | $R^4$ | $R^3$ | $R^2$ | $R^1$ | Schm./°C |
|---|---|---|---|---|---|
| 128 | Cyclopropyl | H | Ethyl | (E)-2-Butenyl | |
| 129 | Cyclopropyl | H | n-Propyl | Ethyl | |
| 130 | Cyclopropyl | H | n-Propyl | Allyl | |
| 131 | Cyclopropyl | H | n-Propyl | (E)-3-Chlor-2-propenyl | |
| 132 | Cyclopropyl | H | n-Propyl | (E)-2-Butenyl | |
| 133 | Cyclohexyl | H | Methyl | Ethyl | |
| 134 | Cyclohexyl | H | Methyl | Allyl | |
| 135 | Cyclohexyl | H | Methyl | (E)-3-Chlor-2-propenyl | |
| 136 | Cyclohexyl | H | Methyl | (E)-2-Butenyl | |
| 137 | Cyclohexyl | H | Ethyl | Ethyl | |
| 138 | Cyclohexyl | H | Ethyl | Allyl | |
| 139 | Cyclohexyl | H | Ethyl | (E)-3-Chlor-2-propenyl | |
| 140 | Cyclohexyl | H | Ethyl | (E)-2-Butenyl | |
| 141 | Cyclohexyl | H | n-Propyl | Ethyl | |
| 142 | Cyclohexyl | H | n-Propyl | Allyl | |
| 143 | Cyclohexyl | H | n-Propyl | (E)-3-Chlor-2-propenyl | |
| 144 | Cyclohexyl | H | n-Propyl | (E)-2-Butenyl | |
| 145 | Methoxymethyl | H | Methyl | Ethyl | |
| 146 | Methoxymethyl | H | Methyl | Allyl | |
| 147 | Methoxymethyl | H | Methyl | (E)-3-Chlor-2-propenyl | |
| 148 | Methoxymethyl | H | Methyl | (E)-2-Butenyl | |
| 149 | Methoxymethyl | H | Ethyl | Ethyl | |
| 150 | Methoxymethyl | H | Ethyl | Allyl | |
| 151 | Methoxymethyl | H | Ethyl | (E)-3-Chlor-2-propenyl | |
| 152 | Methoxymethyl | H | Ethyl | (E)-2-Butenyl | |
| 153 | Methoxymethyl | H | n-Propyl | Ethyl | |
| 154 | Methoxymethyl | H | n-Propyl | Allyl | |
| 155 | Methoxymethyl | H | n-Propyl | (E)-3-Chlor-2-propenyl | |

| | $R^4$ | $R^3$ | $R^2$ | $R^1$ | Schm./°C |
|---|---|---|---|---|---|
| 156 | Methoxymethyl | H | n-Propyl | (E)-2-Butenyl | |
| 157 | Ethoxymethyl | H | Methyl | Ethyl | |
| 158 | Ethoxymethyl | H | Methyl | Allyl | |
| 159 | Ethoxymethyl | H | Methyl | (E)-3-Chlor-2-propenyl | |
| 160 | Ethoxymethyl | H | Methyl | (E)-2-Butenyl | |
| 161 | Ethoxymethyl | H | Ethyl | Ethyl | |
| 162 | Ethoxymethyl | H | Ethyl | Allyl | |
| 163 | Ethoxymethyl | H | Ethyl | (E)-3-Chlor-2-propenyl | |
| 164 | Ethoxymethyl | H | Ethyl | (E)-2-Butenyl | |
| 165 | Ethoxymethyl | H | n-Propyl | Ethyl | |
| 166 | Ethoxymethyl | H | n-Propyl | Allyl | |
| 167 | Ethoxymethyl | H | n-Propyl | (E)-3-Chlor-2-propenyl | |
| 168 | Ethoxymethyl | H | n-Propyl | (E)-2-Butenyl | |
| 169 | Methylthiomethyl | H | Methyl | Ethyl | |
| 170 | Methylthiomethyl | H | Methyl | Allyl | |
| 171 | Methylthiomethyl | H | Methyl | (E)-3-Chlor-2-propenyl | |
| 172 | Methylthiomethyl | H | Methyl | (E)-2-Butenyl | |
| 173 | Methylthiomethyl | H | Ethyl | Ethyl | |
| 174 | Methylthiomethyl | H | Ethyl | Allyl | |
| 175 | Methylthiomethyl | H | Ethyl | (E)-3-Chlor-2-propenyl | |
| 176 | Methylthiomethyl | H | Ethyl | (E)-2-Butenyl | |
| 177 | Methylthiomethyl | H | n-Propyl | Ethyl | |
| 178 | Methylthiomethyl | H | n-Propyl | Allyl | |
| 179 | Methylthiomethyl | H | n-Propyl | (E)-3-Chlor-2-propenyl | |
| 180 | Methylthiomethyl | H | n-Propyl | (E)-2-Butenyl | |
| 181 | Ethylthiomethyl | H | Methyl | Ethyl | |
| 182 | Ethylthiomethyl | H | Methyl | Allyl | |
| 183 | Ethylthiomethyl | H | Methyl | (E)-3-Chlor-2-propenyl | |
| 184 | Ethylthiomethyl | H | Methyl | (E)-2-Butenyl | |

| | $R^4$ | $R^3$ | $R^2$ | $R^1$ | Schm./°C |
|---|---|---|---|---|---|
| 185 | Ethylthiomethyl | H | Ethyl | Ethyl | |
| 186 | Ethylthiomethyl | H | Ethyl | Allyl | |
| 187 | Ethylthiomethyl | H | Ethyl | (E)-3-Chlor-2-propenyl | |
| 188 | Ethylthiomethyl | H | Ethyl | (E)-2-Butenyl | |
| 189 | Ethylthiomethyl | H | n-Propyl | Ethyl | |
| 190 | Ethylthiomethyl | H | n-Propyl | Allyl | |
| 191 | Ethylthiomethyl | H | n-Propyl | (E)-3-Chlor-2-propenyl | |
| 192 | Ethylthiomethyl | H | n-Propyl | (E)-2-Butenyl | |
| 193 | 1-Methoxyethyl | H | Methyl | Ethyl | |
| 194 | 1-Methoxyethyl | H | Methyl | Allyl | |
| 195 | 1-Methoxyethyl | H | Methyl | (E)-3-Chlor-2-propenyl | |
| 196 | 1-Methoxyethyl | H | Methyl | (E)-2-Butenyl | |
| 197 | 1-Methoxyethyl | H | Ethyl | Ethyl | |
| 198 | 1-Methoxyethyl | H | Ethyl | Allyl | |
| 199 | 1-Methoxyethyl | H | Ethyl | (E)-3-Chlor-2-propenyl | |
| 200 | 1-Methoxyethyl | H | Ethyl | (E)-2-Butenyl | |
| 201 | 1-Methoxyethyl | H | n-Propyl | Ethyl | |
| 202 | 1-Methoxyethyl | H | n-Propyl | Allyl | |
| 203 | 1-Methoxyethyl | H | n-Propyl | (E)-3-Chlor-2-propenyl | |
| 204 | 1-Methoxyethyl | H | n-Propyl | (E)-2-Butenyl | |
| 205 | 2-Methylthioethyl | H | Methyl | Ethyl | |
| 206 | 2-Methylthioethyl | H | Methyl | Allyl | |
| 207 | 2-Methylthioethyl | H | Methyl | (E)-3-Chlor-2-propenyl | |
| 208 | 2-Methylthioethyl | H | Methyl | (E)-2-Butenyl | |
| 209 | 2-Methylthioethyl | H | Ethyl | Ethyl | |
| 210 | 2-Methylthioethyl | H | Ethyl | Allyl | |

| | $R^4$ | $R^3$ | $R^2$ | $R^1$ | Schm./°C |
|---|---|---|---|---|---|
| 211 | 2-Methylthioethyl | H | Ethyl | (E)-3-Chlor-2-propenyl | |
| 212 | 2-Methylthioethyl | H | Ethyl | (E)-2-Butenyl | |
| 213 | 2-Methylthioethyl | H | n-Propyl | Ethyl | |
| 214 | 2-Methylthioethyl | H | n-Propyl | Allyl | |
| 215 | 2-Methylthioethyl | H | n-Propyl | (E)-3-Chlor-2-propenyl | |
| 216 | 2-Methylthioethyl | H | n-Propyl | (E)-2-Butenyl | |
| 217 | 1,3-Dimethoxypropyl | H | Methyl | Ethyl | |
| 218 | 1,3-Dimethoxypropyl | H | Methyl | Allyl | |
| 219 | 1,3-Dimethoxypropyl | H | Methyl | (E)-3-Chlor-2-propenyl | |
| 220 | 1,3-Dimethoxypropyl | H | Methyl | (E)-2-Butenyl | |
| 221 | 1,3-Dimethoxypropyl | H | Ethyl | Ethyl | |
| 222 | 1,3-Dimethoxypropyl | H | Ethyl | Allyl | |
| 223 | 1,3-Dimethoxypropyl | H | Ethyl | (E)-3-Chlor-2-propenyl | |
| 224 | 1,3-Dimethoxypropyl | H | Ethyl | (E)-2-Butenyl | |
| 225 | 1,3-Dimethoxypropyl | H | n-Propyl | Ethyl | |
| 226 | 1,3-Dimethoxypropyl | H | n-Propyl | Allyl | |
| 227 | 1,3-Dimethoxypropyl | H | n-Propyl | (E)-3-Chlor-2-propenyl | |
| 228 | 1,3-Dimethoxypropyl | H | n-Propyl | (E)-2-Butenyl | |
| 229 | 2-Ethoxyethyl | H | Methyl | Ethyl | |
| 230 | 2-Ethoxyethyl | H | Methyl | Allyl | |
| 231 | 2-Ethoxyethyl | H | Methyl | (E)-3-Chlor-2-propenyl | |
| 232 | 2-Ethoxyethyl | H | Methyl | (E)-2-Butenyl | |
| 233 | 2-Ethoxyethyl | H | Ethyl | Ethyl | |
| 234 | 2-Ethoxyethyl | H | Ethyl | Allyl | |
| 235 | 2-Ethoxyethyl | H | Ethyl | (E)-3-Chlor-2-propenyl | |
| 236 | 2-Ethoxyethyl | H | Ethyl | (E)-2-Butenyl | |
| 237 | 2-Ethoxyethyl | H | n-Propyl | Ethyl | |
| 238 | 2-Ethoxyethyl | H | n-Propyl | Allyl | |
| 239 | 2-Ethoxyethyl | H | n-Propyl | (E)-3-Chlor-2-propenyl | |

| | $R^4$ | $R^3$ | $R^2$ | $R^1$ | Schm./°C |
|---|---|---|---|---|---|
| 240 | 2-Ethoxyethyl | H | n-Propyl | (E)-2-Butenyl | |
| 241 | 1-Methyl-2-methyl-thioethyl | H | Methyl | Ethyl | |
| 242 | 1-Methyl-2-methyl-thioethyl | H | Methyl | Allyl | |
| 243 | 1-Methyl-2-methyl-thioethyl | H | Methyl | (E)-3-Chlor-2-propenyl | |
| 244 | 1-Methyl-2-methyl-thioethyl | H | Methyl | (E)-2-Butenyl | |
| 245 | 1-Methyl-2-methyl-thioethyl | H | Ethyl | Ethyl | |
| 246 | 1-Methyl-2-methyl-thioethyl | H | Ethyl | Allyl | |
| 247 | 1-Methyl-2-methyl-thioethyl | H | Ethyl | (E)-3-Chlor-2-propenyl | |
| 248 | 1-Methyl-2-methyl-thioethyl | H | Ethyl | (E)-2-Butenyl | |
| 249 | 1-Methyl-2-methyl-thioethyl | H | n-Propyl | Ethyl | |
| 250 | 1-Methyl-2-methyl-thioethyl | H | n-Propyl | Allyl | |
| 251 | 1-Methyl-2-methyl-thioethyl | H | n-Propyl | (E)-3-Chlor-2-propenyl | |
| 252 | 1-Methyl-2-methyl-thioethyl | H | n-Propyl | (E)-2-Butenyl | |
| 253 | 2-Methyl-1-methyl-thiomethyl-propyl | H | Methyl | Ethyl | |
| 254 | 2-Methyl-1-methyl-thiomethyl-propyl | H | Methyl | Allyl | |
| 255 | 2-Methyl-1-methyl-thiomethyl-propyl | H | Methyl | (E)-3-Chlor-2-propenyl | |
| 256 | 2-Methyl-1-methyl-thiomethyl-propyl | H | Methyl | (E)-2-Butenyl | |

| | $R^4$ | $R^3$ | $R^2$ | $R^1$ | Schm./°C |
|---|---|---|---|---|---|
| 257 | 2-Methyl-1-methyl-thiomethyl-propyl | H | Ethyl | Ethyl | |
| 258 | 2-Methyl-1-methyl-thiomethyl-propyl | H | Ethyl | Allyl | |
| 259 | 2-Methyl-1-methyl-thiomethyl-propyl | H | Ethyl | (E)-3-Chlor-2-propenyl | |
| 260 | 2-Methyl-1-methyl-thiomethyl-propyl | H | Ethyl | (E)-2-Butenyl | |
| 261 | 2-Methyl-1-methyl-thiomethyl-propyl | H | n-Propyl | Ethyl | |
| 262 | 2-Methyl-1-methyl-thiomethyl-propyl | H | n-Propyl | Allyl | |
| 263 | 2-Methyl-1-methyl-thiomethyl-propyl | H | n-Propyl | (E)-3-Chlor-2-propenyl | |
| 264 | 2-Methyl-1-methyl-thiomethyl-propyl | H | n-Propyl | (E)-2-Butenyl | |
| 265 | 1-Methylthio-methyl-butyl | H | Methyl | Ethyl | |
| 266 | 1-Methylthio-methyl-butyl | H | Methyl | Allyl | |
| 267 | 1-Methylthio-methyl-butyl | H | Methyl | (E)-3-Chlor-2-propenyl | |
| 268 | 1-Methylthio-methyl-butyl | H | Methyl | (E)-2-Butenyl | |
| 269 | 1-Methylthio-methyl-butyl | H | Ethyl | Ethyl | |
| 270 | 1-Methylthio-methyl-butyl | H | Ethyl | Allyl | |
| 271 | 1-Methylthio-methyl-butyl | H | Ethyl | (E)-3-Chlor-2-propenyl | |
| 272 | 1-Methylthio-methyl-butyl | H | Ethyl | (E)-2-Butenyl | |

| | $R^4$ | $R^3$ | $R^2$ | $R^1$ | Schm./°C |
|---|---|---|---|---|---|
| 273 | 1-Methylthio-methyl-butyl | H | n-Propyl | Ethyl | |
| 274 | 1-Methylthio-methyl-butyl | H | n-Propyl | Allyl | |
| 275 | 1-Methylthio-methyl-butyl | H | n-Propyl | (E)-3-Chlor-2-propenyl | |
| 276 | 1-Methylthio-methyl-butyl | H | n-Propyl | (E)-2-Butenyl | |
| 277 | 2-Methyl-1-propenyl | H | Methyl | Ethyl | |
| 278 | 2-Methyl-1-propenyl | H | Methyl | Allyl | |
| 279 | 2-Methyl-1-propenyl | H | Methyl | (E)-3-Chlor-2-propenyl | |
| 280 | 2-Methyl-1-propenyl | H | Methyl | (E)-2-Butenyl | |
| 281 | 2-Methyl-1-propenyl | H | Ethyl | Ethyl | |
| 282 | 2-Methyl-1-propenyl | H | Ethyl | Allyl | |
| 283 | 2-Methyl-1-propenyl | H | Ethyl | (E)-3-Chlor-2-propenyl | |
| 284 | 2-Methyl-1-propenyl | H | Ethyl | (E)-2-Butenyl | |
| 285 | 2-Methyl-1-propenyl | H | n-Propyl | Ethyl | |
| 286 | 2-Methyl-1-propenyl | H | n-Propyl | Allyl | |
| 287 | 2-Methyl-1-propenyl | H | n-Propyl | (E)-3-Chlor-2-propenyl | |
| 288 | 2-Methyl-1-propenyl | H | n-Propyl | (E)-2-Butenyl | |
| 289 | Methoxymethyl | H | n-Propyl | 3-Methyl-5-isoxazolylmethyl | |
| 290 | Methoxymethyl | H | n-Propyl | 5-Chlor-3-thenyl | |
| 291 | Methoxymethyl | H | n-Propyl | 3-Thenyl | |
| 292 | i-Propyl | H | Ethyl | 5-Chlor-2-thenyl | 80- 84 |
| 293 | i-Propyl | H | Ethyl | 5-Brom-2-thenyl | 79- 84 |
| 294 | i-Propyl | H | n-Propyl | 5-Chlor-2-thenyl | 82- 87 |
| 295 | i-Propyl | H | n-Propyl | 5-Brom-2-thenyl | 86- 90 |
| 296 | i-Propyl | H | n-Propyl | 3-Chlorbenzyl | 72- 74 |
| 297 | i-Propyl | H | n-Propyl | 4-Cyanbenzyl | 108-112 |
| 298 | i-Propyl | H | n-Propyl | 3-Methyl-5-isoxazolylmethyl | 76- 79 |
| 299 | i-Propyl | H | n-Propyl | 3,3-Dichlorcyclopropylmethyl | 89- 91 |

| | $R^4$ | $R^3$ | $R^2$ | $R^1$ | Schm./°C |
|---|---|---|---|---|---|
| 300 | i-Propyl | H | n-Propyl | 4-Chlorbenzyl | 113-114 |
| 301 | i-Propyl | H | n-Propyl | 3-Trifluormethylbenzyl | 122-123 |
| 302 | i-Propyl | H | Ethyl | 4-Chlorbenzyl | 106-109 |
| 303 | i-Propyl | H | Ethyl | 3-Trifluormethylbenzyl | 75- 76 |
| 304 | t-Butyl | H | n-Propyl | 2-Chlorphenetyl | Öl |
| 305 | Ethyl | H | Ethyl | 2-Chlorphenetyl | Öl |
| 306 | i-Propyl | H | Ethyl | 2-Chlorphenetyl | Öl |
| 307 | i-Propyl | H | Methyl | 2-Chlorphenetyl | Öl |
| 308 | 1-Methoxyethyl | Benzoyl | n-Propyl | Ethyl | Öl |
| 309 | 1-Methoxyethyl | Propionyl | n-Propyl | Ethyl | Öl |
| 310 | 1-Methoxyethyl | Butyryl | n-Propyl | Ethyl | Öl |
| 311 | 1-Methoxyethyl | Pivaloyl | n-Propyl | Ethyl | Öl |
| 312 | 2,6-Dimethylheptyl | Propionyl | Ethyl | Allyl | Öl |
| 313 | 2,6-Dimethylheptyl | Propionyl | Ethyl | Allyl | Öl |
| 314 | 2,6-Dimethylheptyl | Benzoyl | Ethyl | Allyl | Öl |
| 315 | 2,6-Dimethylheptyl | Pivaloyl | Ethyl | Allyl | Öl |
| 316 | 2,6-Dimethylheptyl | Butyryl | n-Propyl | Ethyl | Öl |
| 317 | 2,6-Dimethylheptyl | Propionyl | n-Propyl | Ethyl | Öl |
| 318 | 2,6-Dimethylheptyl | Benzoyl | n-Propyl | Ethyl | Öl |
| 319 | 2,6-Dimethylheptyl | Palmitoyl | n-Propyl | Ethyl | Öl |
| 320 | 1-Methoxyethyl | Butyryl | Ethyl | Allyl | Öl |
| 321 | 1-Methoxyethyl | Benzoyl | Ethyl | Allyl | Öl |
| 322 | 1-Methoxyethyl | Pivaloyl | Ethyl | Allyl | Öl |
| 323 | 1-Methoxyethyl | Palmitoyl | Ethyl | Allyl | Öl |
| 324 | 1-Methoxyethoxyethyl | H | n-Propyl | Ethyl | |
| 325 | 1-Methoxyethoxyethyl | H | n-Propyl | Allyl | |
| 326 | 1-Methoxyethoxyethyl | H | n-Propyl | (E)-2-Butenyl | |
| 327 | 1-Methoxyethoxyethyl | H | n-Propyl | (E)-3-Chlor-2-propenyl | |
| 328 | 1-Methoxymethyl-ethyl | H | Ethyl | Ethyl | |

| | $R^4$ | $R^3$ | $R^2$ | $R^1$ | Schm./°C |
|---|---|---|---|---|---|
| 329 | 1-Methoxymethyl-ethyl | H | Ethyl | Allyl | |
| 330 | 1-Methoxymethyl-ethyl | H | Ethyl | (E)-2-Butenyl | |
| 331 | 1-Methoxymethyl-ethyl | H | Ethyl | (E-3-Chlor-2-propenyl | |
| 332 | 1-Methoxymethyl-ethyl | H | n-Propyl | Ethyl | |
| 333 | 1-Methoxymethyl-ethyl | H | n-Propyl | Allyl | |
| 334 | 1-Methoxymethyl-ethyl | H | n-Propyl | (E)-2-Butenyl | |
| 335 | 1-Methoxymethyl-ethyl | H | n-Propyl | (E)-3-Chlor-2-propenyl | |
| 336 | i-Propyl | Butyryl | Ethyl | (E)-2-Butenyl | Öl |
| 337 | i-Propyl | Pivaloyl | Ethyl | (E)-2-Butenyl | Öl |
| 338 | i-Propyl | Benzoyl | Ethyl | (E)-2-Butenyl | Öl |
| 339 | i-Propyl | Butyryl | Ethyl | Ethyl | Öl |
| 341 | i-Propyl | Pivaloyl | Ethyl | Ethyl | Öl |
| 342 | i-Propyl | Propionyl | Ethyl | (E)-3-Chlor-2-propenyl | Öl |
| 343 | i-Propyl | Pivaloyl | Ethyl | (E)-3-Chlor-2-propenyl | Öl |
| 344 | i-Propyl | Na | Ethyl | Ethyl | |
| 345 | i-Propyl | $NH_4$ | Ethyl | Ethyl | |
| 346 | i-Propyl | $^1/_2$ Fe | Ethyl | Ethyl | |
| 347 | i-Propyl | $^1/_2$ Mg | Ethyl | Ethyl | |

Tabelle 2 - Elementaranalysen und $^1H$-NMR-Daten ausgewählter Wirkstoffe

| Wirkstoff No. | $^1H$-NMR-Daten |
|---|---|
| 5 | 1,16 (t,3H), 1,26 (t,3H), 1,34 (t, 3H), 2,68 (q, 2H), 4,13 (q,2H), 5,92 (s, 1H) |
| 6 | 1,16 (t,3H), 1,28 (t,3H), 2,78 (q,2H), 4,56 (d,2H), 5,92 (s,1H) |
| 7 | 1,13 (t,3H), 1,25 (t,3H), 2,66 (q,2H), 4,52 (d,2H), 5,9 (s,1H), 6,35 (d,1H) |
| 8 | 1,13 (t,3H), 1,26 (t,3H), 1,78 (d,3H), 2,68 (q,2H), 4,45 (d,2H), 5,9 (s,1H) |
| 9 | 0,96 (t,3H), 1,24 (t,3H), 1,3 (t,3H), 1,54 ("6",2H), 4,1 (q,2H), 5,92 (s,1H) |
| 10 | 0,96 (t,3H), 1,24 (t, 3H), 1,54 ("6",2H), 2,65 (q,2H), 4,52 (d,2H), 5,9 (s,1H) |
| 11 | 0,95 (t,3H), 1,25 (t,3H), 2,65 (q,2H), 4,5 (d,2H), 5,92 (s,1H), 6,08 (dt,1H), 6,35 (d,1H) |
| 12 | 0,95 (t,3H), 1,25 (t,3H), 1,54 ("6",2H), 1,75 (d,3H), 2,65 (q,2H), 4,45 (d,2H), 5,92 (s,1H) |
| 17 | 0,97 (t,3H), 1,13 (t,3H), 1,34 (t,3H), 1,67 ("6",2H), 2,6 (t,2H), 4,12 (q,2H), 5,9 (s,1H) |
| 18 | 0,97 (t,3H), 1,14 (t,3H), 1,67 ("6",2H), 2,6 (t,2H), 4,53 (d,2H), 5,36 (dd,2H), 5,9 (s,1H) |
| 19 | 0,97 (t,3H), 1,13 (t,3H), 166 ("6",2H), 4,52 ((d,2H), 5,9 (s,1H), 6,35 (d,1H) |
| 20 | 0,97 (t,3H), 1,14 (t,3H), 2,6 (t,2H), 4,47 (d,2H), 5,9 (s,1H) |

Tabelle 2 - Elementaranalysen und $^1$H-NMR-Daten ausgewählter Wirkstoffe

| Wirkstoff No. | $^1$H-NMR-Daten |
|---|---|
| 21 | 0,96 (t,6H), 1,14 (t,3H), 2,6 (t,2H, 4,11 (q,2H), 5,92 (s,1H) |
| 22 | 0,96 (t,6H), 1,54 ("6",2H), 1,67 ("6",2H), 2,59 (t,2H), 4,53 (d, 2H), 5,9 (s,1H) |
| 23 | 0,95 (t,3H), 0,97 (t,3H), 1,53 ("6",2H), 1,69 ("6",2H), 2,6 (t,2H), 4,53 (d,2H), 5,92 (s,1H), 6,1 (dt,1H), 6,36 (d, 1H) |
| 24 | 0,96 (t,6H), 1,54 ("6",2H), 1,67 ("6",2H), 1,73 (d,3H), 2,6 (t,2H), 4,45 (d,2H), 5,91 (s,1H) |
| 25 | 1,3 (d,6H), 2,34 (s,3H), 4,1 (q,2H), 5,9 (s,1H) |
| 26 | 1,27 (d,6H), 2,39 (s,3H), 4,55 (d,2H), 5,92 (s,1H) |
| 27 | 1,28 (d,6H), 2,32 (s,3H), 4,52 (d,2H), 5,93 (s,1H), 6,34 (d,1H) |

Tabelle 2 - Elementaranalysen und $^1$H-NMR-Daten ausgewählter Wirkstoffe

| Wirkstoff No. | | Elementaranalyse C | H | N | S | Cl | Br | $^1$H-NMR-Daten |
|---|---|---|---|---|---|---|---|---|
| 28 | ber. | 65,0 | 7,3 | 8,4 | | | | 1,25 (d,6H), 1,76 (d,3H), 2,4 (s,3H) |
| | gef. | 64,6 | 7,3 | 8,7 | | | | 4,47 (d,2H), 5,9 (s,1H) |
| 29 | ber. | 63,7 | 7,8 | 8,8 | | | | 1,15 (t,3H), 1,25 (d,6H), 1,3 (t,3H) |
| | gef. | 63,2 | 7,5 | 8,8 | | | | 4,1 (q,2H), 5,92 (s,1H) |
| 30 | ber. | 65,0 | 7,3 | 8,4 | | | | 1,13 (t,3H), 1,25 (d,6H), 4,53 (d,2H) |
| | gef. | 64,8 | 7,3 | 8,7 | | | | 5,92 (s,1H), |
| 31 | ber. | 58,9 | 6,3 | 7,6 | | 9,7 | | 1,12 (t,3H), 1,26 (d,6H), 4,5 (d,2H) |
| | gef. | 59,1 | 6,2 | 7,7 | | 9,3 | | 5,91 (s,1H) |
| 32 | ber. | 65,9 | 7,6 | 8,1 | | | | 1,15 (t,3H), 1,28 (d,6H), 1,78 (d,3H) |
| | gef. | 65,5 | 7,2 | 8,4 | | | | 4,45 (d,2H), 5,93 (s,1H) |
| 33 | ber. | 64,7 | 7,8 | 8,4 | | | | 0,96 (t,3H), 1,27 (d,6H), 1,3 (t,3H) |
| | gef. | 64,3 | 7,7 | 8,5 | | | | 1,55 ("6",2H), 4,1 (q,2H), 6,94 (s,1H) |
| 34 | ber. | 65,9 | 7,6 | 8,1 | | | | 0,96 (t,3H), 1,28 (d,6H), 1,54 ("6",2H), |
| | gef. | 64,4 | 7,5 | 8,7 | | | | 4,52 (d,2H), 5,92 (s,1H) |
| 35 | ber. | 59,9 | 6,6 | 7,4 | | 9,3 | | 0,96 (t,3H), 1,27 (d,6H), 1,53 ("6"),2H), |
| | gef. | 59,3 | 6,5 | 7,6 | | 9,2 | | 4,53 (d,2H), 5,92 (s,1H) |
| 36 | ber. | 66,6 | 7,8 | 7,8 | | | | 0,97 (t,3H), 1,27 (d,6H), 1.53 ("6",2H) |
| | gef. | 66.3 | 7,6 | 7,6 | | | | 1,76 (d,3H), 4,42 (d,2H), 5,91 (s,1H) |

| Wirkstoff No. | Elementaranalyse C | H | N | S | Cl | Br | $^1$H-NMR-Daten |
|---|---|---|---|---|---|---|---|
| 37 | | | | | | | 0,97 (t,3H), 1,37 (t,3H), 1,67 ("5",2H), 2,45 (s,3H), 2,68 (t,2H), 4,17 (q,2H), 5,9 (s,1H) |
| 38 | | | | | | | 0,96 (t,3H), 1,42 ("6",2H), 1,67 ("5",2H), 2,42 (s,3H), 2,67 (t,2H), 4,59 (d,2H), 5,9 (s,1H), 6,4 (d,1H) |
| 39 | | | | | | | 0.97 (t,3H), 1,42 ("6",2H), 1,67 ("5",2H), 2,34 (s,3H), 2,67 (t,2H), 4,6 (d,2H), 5,97 (s,1H), 6,4 (d,1H) |
| 40 | | | | | | | 0,96 (t,3H), 1,41 ("6",2H), 1;66 ("5",2H), 1,8 (d,3H), 2,42 (s,3H), 2,66 (t,2H), 4,48 (d,2H), 5,93 (s,1H) |
| 41 | | | | | | | 0,94 (t,3H), 1,15 (t,3H), 1,33 (t,3H), 1,63 ("5",2H), 2,63 (t,2H), 4,12 (q,2H), 5,91 (s,1H) |
| 42 | | | | | | | 0,94 (t,3H), 4,53 (d,2H), 5,34 (dd,2H), 5,9 (s,1H) |
| 43 | | | | | | | 0,94 (t,3H), 1,12 (t,3H), 1,38 ("6", 2H), 1,64 ("5",2H), 2,63 (t,2H), 4,52 (d,2H), 5,9 (s,1H), 6,35 (d, 1H) |
| 44 | | | | | | | 0,93 (t,3H), 1,14 (t,3H), 1,38 ("6",2H), 1,61 ("5",2H), 1,77 (d,3H), 2,63 (t,2H), 4,46 (d,2H), 5,89 (s,1H) |
| 45 | | | | | | | 0,8 (t,3H), 0,88 (t,3H), 3,88 (q,2H), 4,02 (q,2H), 6,21 (s,1H) |
| 46 | | | | | | | 0,82 (t,3H), 0,88 (t,3H), 4,5 (d,2H), 6,21 (s,1H) |
| 48 | | | | | | | 0,84 (t,3H), 0,92 (t,3H), 1,7 (d,3H), 6,24 (s,1H) |
| 53 | | | | | | | 0,9 (t,3H), 1,15 (t,3H), 1,25 (d,3H), 1,32 (t,3H), 4,15 (q,2H), 5,92 (s,1H) |
| 54 | | | | | | | 0,89 (t,3H), 1,15 (t,3H), 1,25 (d,3H), 4,56 (d,2H), 5,91 (s,1H) |

| Wirkstoff No. | $^1$H-NMR-Daten |
|---|---|
| 55 | 0,9 (t,3H), 1,13 (t,3H), 1,25 (d,3H), 4,56 (d,2H), 5,92 (s,1H), 6,12 (dt,1H), 6,38 (d,1H) |
| 56 | 0,9 (t,3H), 1,15 (t,3H), 1,25 (d,3H), 1,78 (d,3H), 4,48 (d,2H), 5,91 (s,1H) |
| 57 | 0,89 (t,3H), 0,96 (t,3H), 1,24 (d,3H), 1,34 (t,3H), 4,12 (q,2H), 5,9 (s,1H) |
| 58 | 0,88 (t,3H), 0,96 (t,3H), 1,23 (d,3H), 4,54 (d,2H), 5,34 (dd,2H), 5,92 (s,1H) |
| 59 | 0,88 (t,3H), 0,95 (t,3H), 1,23 (d,3H), 4,53 (d,2H), 5,9 (s,1H), 6,1 (dt, 1H), 6,36 (d,1H) |
| 60 | 0,9 (t,3H), 0,95 (t,3H), 1,24 (d,3H), 1,7 (d,3H), 4,43 (d,2H), 5,89 (s,1H) |
| 65 | 1,13 (t,3H), 1,3 (s,9H), 4,1 (q,2H), 5,95 (s, 1H) |
| 66 | 1,16 (t,3H), 1,34 (s,9H), 4,55 (d,2H), 5,9 (s,1H) |
| 67 | 1,1 (t,3H), 1,3 (s,9H), 4,53 (d,2H), 5,93 (s,1H), 6,35 (d,1H) |
| 68 | 1,12 (t,3H), 1,3 (s,9H), 1,77 (d,3H), 4,45 (d,2H), 5,92 (s,1H) |
| 69 | 0,95 (t,3H), 1,32 (s,9H), 1,53 ("6"),ZH), 4,1 (q,2H), 5,97 (s,1H) |
| 70 | 0,96 (t,3H), 1,3 (s,9H), 4,53 (d,2H), 5,92 (s, 1H) |
| 71 | 0,95 (t,3H), 1,3 (s,9H), 1,5 ("6", 2H), 4,52 (d,2H), 5,94 (s, 1H), 6,35 (d, 1H) |
| 72 | 0,95 (t,3H), 1,3 (s,9H), 1,77 (d,3H) 4,44 (d,2H), 5,93 (s,1H) |
| 77 | 0,97 (d,6H), 1,17 (t,3H), 1,34 (t,3H), 2,53 (d,2H), 4,14 (q,2H), 5,9 (s,1H) |
| 78 | 0,98 (d,6H), 1,17 (t,3H), 2,54 (d,2H), 4,57 (d,2H), 5,4 (dd,2H), 5,9 (s,1H) |

| Wirkstoff No. | $^1H$-NMR-Daten |
|---|---|
| 79 | 0,95 (d,6H), 1,13 (t,3H), 2,51 (d,2H), 4,52 (d,2H), 5,9 (s,1H), 6,1 (dt,1H), 6,35 (d, 1H) |
| 80 | 0,95 (d,6H), 1,14 (t,3H), 1,77 (d,3H), 2,5 (d,2H), 4,46 (d,2H), 5,88 (s,1H) |
| 81 | 0,96 (d,6H), 1,32 (t,3H), 1,55 ("6",2H), 2,5 (d,2H), 4,11 (q,2H), 5,88 (s,1H) |
| 82 | 0,95 (d,6H), 0,97 (t,3H), 1,54 ("6",2H), 1,95 ("7",2H), 2,5 (d,2H), 4,54 (d,2H), 5,9 (s, 1H) |
| 83 | 0,95 (d,6H), 1,52 ("6",2H), 1,95 ("7",2H), 2,5 (d,2H), 4,53 (d,2H), 5,9 (s,1H), 6,1 (dt,1H), 6,36 (d,1H) |
| 84 | 0,94 (d,6H), 1,54 ("6",2H), 1,77 (d,3H), 1,95 ("7",2H), 2,5 (d,2H), 4,45 (d,2H), 5,87 (s,1H) |
| 89 | 0,88 (s,9H), 0,98 (d,3H), 1,16 (t,3H), 1,34 (t,3H), 4,12 (q, 2H), 5,9 (s,1H) |
| 90 | 0,87 (s,9H), 0,97 (d,3H), 1,16 (t,3H), 4,55 (d,2H), 5,89 (s,1H) |
| 91 | 0,87 (s,9H), 0,96 (d,3H), 1,13 (t,3H), 4,52 (d,2H), 5,9 (s,1H), 6,1 (dt,1H), 6,35 (d,1H) |
| 92 | 0,87 (s,9H), 0,97 (d,3H), 1,17 (t,3H), 1,77 (d,3H), 4,47 (d,2H), 5,88 (s,1H) |
| 93 | 0,87 (s,9H), 0,97 (d,3H), 1,32 (t,2H), 4,12 (q,2H), 5,88 (s,1H) |
| 94 | 0,87 (s,9H), 0,97 (d,3H), 0,99 (t,3H), 4,53 (d,2H), 5,89 (s,1H) |
| 95 | 0,87 (s,9H), 0,96 (d,3H), 4,53 (d,2H), 5,9 (s,1H), 6,35 (d,1H) |
| 96 | 0,87 (s,9H), 0,96 (d,3H), 1,77 (d,3H), 4,44 (d,2H), 5,88 (s,1H) |

| Wirkstoff No. | $^1$H-NMR-Daten |
|---|---|
| 105 | 0,84 (t,3H), 0,87 (t,3H), 1,14 (t,3H), 1,33 (t,3H), 4,12 (q,2H), 5,88 (s,1H) |
| 106 | 0,83 (t,3H), 0,86 (t,3H), 1,14 (t,3H), 4,56 (d,2H), 5,87 (s,1H) |
| 107 | 0,84 (t,3H), 0,87 (t,3H), 1,12 (t,3H), 4,55 (d,2H), 5,88 (s,1H), 6,11 (dt,1H), 6,37 (d,1H) |
| 108 | 0,83 (t,3H), 0,86 (t,3H), 1,14 (t,3H), 1,78 (d,3H), 4,48 (d,2H), 6,88 (s,1H) |
| 113 | 0,85 (d,6H), 0,87 (t,3H), 1,14 (t,3H), 1,35 (t,3H), 4,12 (q,2H), 5,9 (s,1H) |
| 114 | 0,85 (d,6H), 0,87 (t,3H), 1,15 (t,3H), 4,55 (d,2H), 5,89 (s,1H) |
| 115 | 0,85 (d,6H), 0,87 (t,3H), 4,53 (d,2H), 5,89 (s,1H), 6,36 (d,1H) |
| 116 | 0,86 (d,6H), 0,9 (t,3H), 1,15 (t,3H), 1,78 (d,3H), 4,48 (d,2H), 5,9 (s,1H) |
| 117 | 0,85 (d,6H), 0,87 (t,3H), 1,34 (t,3H), 4,13 (q,2H), 5,9 (s,1H) |
| 118 | 0,85 (d,6H), 0,87 (t,3H), 4,52 (d,2H), 5,36 (dd,2H), 5,89 (s,1H) |
| 119 | 0,87 (d,6H), 0,95 (t,3H), 4,53 (d,2H), 5,9 (s,1H), 6,1 (dt,1H), 6,38 (d,1H) |
| 120 | 0,85 (d,6H), 0,87(t,3H), 0,95 (t,3H) 1,76 (d,3H), 4,45 (d,2H), 5,88 (s,1H) |
| 137 | 1,14 (t,3H), 1,35 (t,3H), 4,1 (q,2H), 5,9 (s,1H) |
| 138 | 1,14 (t,3H), 4,54 (d,2H), 5,35 (dd,2H), 5,9 (s,1H) |
| 139 | 1,12 (t,3H), 4,52 (d,2H), 5,9 (s,1H), 6,1 (dt,1H), 6,35 (d,1H) |

| Wirkstoff No. | | Elementaranalyse C | H | N | S | Cl | Br | $^1$H-NMR-Daten |
|---|---|---|---|---|---|---|---|---|
| 140 | | | | | | | | 1,13 (t,3H), 1,75 (d,3H), 4,45 (d,2H), 5,88 (s,1H) |
| 141 | | | | | | | | 0,96 (t,3H), 1,32 (t,3H), 4,1 (q,2H), 5,9 (s,1H) |
| 142 | | | | | | | | 0,95 (t,3H), 1,55 ("6",2H), 4,54 (d,2H), 5,36 (dd,2H), 5.9 (s,1H) |
| 143 | | | | | | | | 0,95 (t,3H), 4,52 (d,2H), 5,9 (s,1H), 6,1 (dt,1H), 6,35 (d,1H) |
| 144 | | | | | | | | 0,96 (t,3H), 1,54 ("6",2H), 1,75 (d,3H), 4,44 (d,2H), 5,88 (s,1H) |
| 153 | ber. | 60,7 | 7,2 | 8,3 | | | | 0,95 (t,3H), 1,31 (6,3H), 1,53 ("6",2H) |
| | gef. | 61,1 | 7,2 | 8,2 | | | | 3,39 (s,3H), 4,12 (q,2H), 4,5 (s,2H), 6,1 (s,1H), 6,12 (s,1H) |
| 154 | ber. | 62,1 | 6,9 | 8,0 | | | | 0,96 (t,3H), 1,54 ("6",2H), 3,4 (s,3H) |
| | gef. | 62,5 | 7,1 | 8,0 | | | | 4,5 (s,2H), 4,54 (d,2H), 6,15 (s,1H) |
| 155 | ber. | 56,5 | 6,1 | 7,3 | | 9,3 | | 0,94 (t,3H), 1,5 ("6",2H), 3,4 (s,3H), |
| | gef. | 57,7 | 6,1 | 6,9 | | 7,4 | | 4,5 (s,2H), 4,52 (d,2H), 6,13 (s,1H) |
| 156 | ber. | 63,0 | 7,2 | 7,7 | | | | 0,97 (t,3H), 1,55 ("6",2H), 1,68 (d,3H), |
| | gef. | 57,7 | 6,1 | 6,9 | | | | 4,5 (s,2H), 4,52 (d,2H), 6,13 (s,1H) |
| 197 | | | | | | | | 1,13 (t,3H), 1,34 (t,3H), 1,47 (d,3H), 3,28 (s,3H), 4,13 (q, 2H), 4,5 (q, 1H), 6,1 (s, 1H) |
| 198 | | | | | | | | 1,13 (t,3H), 1,46 (d,3H), 3,27 (s,3H), 4,5 (q,1H), 4,55 (d,2H), 5,35 (dd,2H), 6,08 (s,1H) |
| 199 | | | | | | | | 1,13 (t,3H), 1,48 (d,3H), 3,28 (s,3H), 4,5 (q,1H), 4,55 (d,2H), 6,08 (s,1H), 6,35 (d,1H) |

| Wirkstoff No. | Elementaranalyse C | H | N | S | Cl | Br | $^1$H-NMR-Daten |
|---|---|---|---|---|---|---|---|
| 200 | | | | | | | 1,14 (t,3H), 1,47 (d,3H), 1,78 (s,3H), 3,28 (s,3H), 4,48 (d,2H), 4,5 (q,1H), 6,08 (s,1H) |
| 201 | | | | | | | 0,97 (t,3H), 1,32 (t,3H), 1,47 (d,3H), 3,3 (s,3H), 4,12 (q,2H), 4,5 (q,1H), 6,08 (s,1H) |
| 202 | | | | | | | 0,96 (t,3H), 1,47 (d,3H), 3,28 (s,3H), 4,5 (q,1H), 4,52 (d, 2H), 5,37 (dd,2H), 6,08 (s,1H) |
| 203 | | | | | | | 0,96 (t,3H), 1,47 (d,3H), 3,3 (s,3H), 4,5 (d,2H), 6,08 (s,1H), 6,34 (d,1H) |
| 204 | | | | | | | 0,97 (t,3H), 1,47 (d,3H), 1,77 (d,3H), 3,28 (s,3H), 4,45 (d,2H), 6,08 (s,1H) |
| 281 | | | | | | | 1,15 (t,3H), 1,32 (t,3H), 1,94 (s,3H), 2,0 (s,3H), 4,12 (q,2H), 6,03 (s,1H), 6,08 (s,1H) |
| 282 | | | | | | | 1,14 (t,3H), 1,94 (s,3H), 2,0 (s,3H), 4,53 (d,2H), 5,37 (dd,2H), 6,01 (s,1H), 6,08 (s,1H) |
| 283 | | | | | | | 1,13 (t,3H), 1,93 (s,3H), 2,0 (s,3H), 4,53 (d,2H), 6,03 (s,1H), 6,07 (s,1H), 6,36 (d,1H) |
| 284 | | | | | | | 1,14 (t,3H), 1,77 (d,3H), 1,93 (s,3H), 2,0 (s,3H), 4,45 (d,2H), 6,02 (s,1H), 6,08 (s,1H) |
| 285 | | | | | | | 0,97 (t,3H), 1,33 (t,3H), 1,55 ("6",2H), 1,93 (s,3H), 2,0 (s,3H), 4,12 (q,2H), 6,02 (s,1H), 6,08 (s,1H) |
| 286 | | | | | | | 0,96 (t,3H), 1,55 ("6",2H), 1,94 (s,3H), 2,0 (s,3H), 4,52 (d,2H), 5,36 (dd,2H), 6,0 (s,1H), 6,08 (s,1H) |

| Wirkstoff No. | | Elementaranalyse C | H | N | S | Cl | Br | $^1$H-NMR-Daten |
|---|---|---|---|---|---|---|---|---|
| 288 | | | | | | | | 0,96 (t,3H), 1,52 ("6",2H), 1,77 (d,3H), 1,95 (s,3H), 1,99 (s,3H), 4,45 (d,2H), 6,0 (s,1H), 6,08 (s,1H) |
| 289 | ber. | 59,5 | 6,3 | 10,4 | | | | 0,93 (t,3H), 1,5 ("6",2H), 2,33 (s,3H) |
| | gef. | 59,8 | 6,2 | 9,7 | | | | 3,4 (s,3H), 4,58 (s,2H), 5,1 (s,2H), 6,14 (s,1H), 6,18 (s,1H) |
| 290 | ber. | 54,7 | 5,3 | 6,4 | 7,3 | 8,1 | | 0,95 (t,3H), 1,5 ("6",2H), 3,4 (s,3H) |
| | gef. | 54,8 | 5,4 | 6,4 | 8,3 | 7,7 | | 4,48 (s,2H), 4,93 (s,2H), 6,1 (s,1H), 6,9 (d,1H), 7,08 (d,1H) |
| 291 | ber. | 59,4 | 6,0 | 6,9 | 7,9 | | | 0,95 (t,3H), 1,5 ("6",2H), 3,38 (s,3H), |
| | gef. | 59,9 | 6,3 | 6,9 | 7,3 | | | 4,48 (s,2H), 5,08 (s,2H), 6,1 (s,1H) |
| 292 | ber. | 56,8 | 5,5 | 6,6 | 7,6 | 8,4 | | 1,12 (t,3H), 1,28 (d,6H), 5,1 (s,2H), |
| | gef. | 56,5 | 5,4 | 7,2 | 7,6 | 8,8 | | 5,92 (s,1H), 6,84 (d,1H), 6,87 (d,1H), |
| 293 | ber. | 51,4 | 4,9 | 6,0 | 6,9 | | 17,1 | 1,1 (t,3H), 1,27 (d,6H), 5,13 (s,2H), |
| | gef. | 51,8 | 5,0 | 6,6 | 6,5 | | 15,8 | 5,92 (s,1H), 6,85 (d,1H), 6,95 (d,1H), |
| 294 | ber. | 57,7 | 5,8 | 6,4 | 7,3 | 8,1 | | 0,93 (t,3H), 1,24 (d,6H), 5,1 (s,2H), |
| | gef. | 57,4 | 5,7 | 7,4 | 7,0 | 7,8 | | 5,95 (s,1H), 6,8 (d,1H), 6,86 (d,1H) |
| 295 | ber. | 52,4 | 5,2 | 5,8 | 6,7 | | 16,6 | 0,92 (t,3H), 1,27 (d,6H), 5,12 (s,2H), |
| | gef. | 51,7 | 5,2 | 6,2 | 6,9 | | 16,6 | 5,93 (s,1H), 6,85 (d,1H), 6,95 (d,1H) |
| 296 | ber. | 64,1 | 6,3 | 6,5 | | 8,2 | | 0,95 (t,3H), 1,25 (d,6H), 5,02 (s,2H), |
| | gef. | 63,8 | 6,4 | 6,9 | | 8,6 | | 5,95 (s,1H), 7,05-7,4 (m,4H) |
| 297 | ber. | 68,4 | 6,5 | 10,0 | | | | 0,95 (t,3H), 1,26 (d,6H), 5,13 (s,2H), |
| | gef. | 68,0 | 6,5 | 10,1 | | | | 5,95 (s,1H), 7,56 (AA'BB',4H) |

| Wirkstoff No. | | Elementaranalyse C | H | N | S | Cl | Br | $^1$H-NMR-Daten |
|---|---|---|---|---|---|---|---|---|
| 298 | ber. | 62,8 | 6,8 | 10,5 | | | | 0,78 (t,3H), 1,2 (d,6H), 2,23 (s,3H), 2,35 (t,2H), 5,1 (s,2H), 6,25 (s,1H), 6,35 (s,1H) |
| | gef. | 62,3 | 6,7 | 10,8 | | | | |
| 299 | ber. | 56,0 | 6,1 | 6,5 | | 16,5 | | 0,98 (t,3H), 1,25 (d,6H), 1,55 ("q",2H), 4,2 (d,2H), 5,93 (s,1H) |
| | gef. | 55,4 | 6,2 | 6,9 | | 16,9 | | |
| 300 | | | | | | | | 0,94 (t,3H), 1,25 (d,6H), 1,52 ("6",2H), 5,0 (s,2H), 5,9 (s,1H), 7,32 (AA'BB',4H) |
| 301 | | | | | | | | 0,94 (t,3H), 1,25 (d,6H), 1,52 ("6",2H), 5,12 (s,2H), 5,9 (s,1H) |
| 302 | | | | | | | | 0,84 (t,3H), 1,17 (d,6H), 5,07 (s,2H), 6,27 (s,1H) |
| 303 | | | | | | | | 0,88 (t,3H), 1,18 (d,6H), 5,17 (s,2H), 6,26 (s,1H) |
| 304 | | | | | | | | 0,9 (t,3H), 1,31 (s,9H), 3,17 (t,2H), 5,96 (s,1H) |
| 305 | | | | | | | | 1,04 (t,3H), 1,23 (t,3H), 2,64 (q,2H), 3,12 (t,2H), 4,27 (t,2H), 5,92 (s,1H) |
| 306 | | | | | | | | 1,08 (t,3H), 1,28 (d,6H), 3,17 (t,2H), 4,3 (t,2H), 5,93 (s,1H) |
| 307 | | | | | | | | 1,27 (d,6H), 2,33 (s,3H), 3,16 (t,2H), 4,32 (t,2H), 5,93 (s,1H) |
| 308 | | | | | | | | 0,92 (t,3H), 1,12 (t,3H), 1,5 (d,3H), 3,33 (s,3H), 4,0 (q,2H), 6,2 (s,1H), 7,53 (t,2H), 7,7 (t,1H), 8,07 (d,2H) |
| 309 | | | | | | | | 0,98 (t,3H), 1,37 (t,3H), 1,5 (d,3H), 3,34 (s,3H), 4,15 (q,2H), 6,12 (s,1H) |
| 310 | | | | | | | | 1,47 (d,3H), 3,28 (s,3H), 6,15 (s,1H) |

| Wirkstoff No. | Elementaranalyse C | H | N | S | Cl | Br | $^1$H-NMR-Daten |
|---|---|---|---|---|---|---|---|
| 311 | | | | | | | 0,9 (t,3H), 1,25 (s,9H), 1,47 (d,3H), 3,3 (s,3H), 4,13 (q,2H), 4,5 (q,1H), 6,16 (s,1H) |
| 312 | | | | | | | 0,88 (d,6H), 4,57 (d,2H), 5,9 (s,1H) |
| 313 | | | | | | | 0,88 (d,6H), 5,94 (s,1H) |
| 314 | | | | | | | 8,88 (d,6H), 4,42 (d,2H), 5,98 (s,1H) |
| 315 | | | | | | | 0,88 (d,6H), 1,25 (s,9H), 4,57 (d,2H), 5,92 (s,1H) |
| 316 | | | | | | | 0,88 (d,6H), 4,13 (q,2H), 5,94 (s,1H) |
| 317 | | | | | | | 0,88 (d,6H), 0,92 (t,3H), 4,12 (q,2H), 5,94 (s,1H) |
| 318 | | | | | | | 0,87 (d,6H), 4,1 (q,2H), 5,88 (s,1H) |
| 320 | | | | | | | 1,45 (d,3H), 3,27 (s,3H), 6,13 (s,1H) |
| 321 | | | | | | | 0,97 (t,3H), 1,46 (d,3H), 3,3 (s,3H), 6,18 (s,1H), 7,5 (t,2H), 7,68 (t,1H), 8,05 (d,2H) |
| 322 | | | | | | | 0,97 (t,3H), 1,28 (s,9H), 1,48 (d,3H), 3,3 (s,3H), 6,13 (s,1H) |
| 323 | | | | | | | 0,91 (t,3H), 3,32 (s,3H), 6,18 (s,1H) |
| 336 | | | | | | | 0,9 (t,3H), 1,0 (t,3H), 1,25 (d,6H), 4,5 (d,2H), 6,0 (s,1H) |
| 337 | | | | | | | 0,91 (t,3H), 1,25 (s,9H), 1,27 (d,6H), 1,72 (d,3H), 6,0 (s,1H) |
| 338 | | | | | | | 0,94 (t,3H), 1,27 (d,6H), 1,54 (d,3H), 6,04 (s,1H), 7,5 (t,2H), 8,03 (t,2H) |
| 339 | | | | | | | 0,88 (t,3H), 1,0 (t,3H), 1,28 (d,6H), 1,7 ("6",2H), 4,12 (q,2H), 5,98 (s,1H) |
| 340 | | | | | | | 0,96 (t,3H), 1,27 (d,6H), 3,98 (q,2H), 6,03 (s,1H) |
| 341 | | | | | | | 0,91 (t,3H), 1,25 (s,9H, 1,27 (d,6H), 4,1 (q,2H), 5,98 (s,1H) |

Die Anwendung der Mittel kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen - (post-directed, lay-by).

Die Aufwandmengen an Wirkstoff betragen je nach Jahreszeit, Pflanzenart und Wachstumsstadium 0,01 bis 3 kg/ha, vorzugsweise 0,05 bis 1,0 kg/ha.

Die neuen Wirkstoffe werden beispielsweise in Form von direkt versprühbaren Lösungen, Suspensionen, Emulsionen, auch in Form von hochprozentigen wäßrigen, öligen oder sonstigen Dispersionen, Pasten, Stäubemitteln, Streumitteln, Granulaten durch Versprühen, Verstäuben, Verstreuen, Verstreichen oder Gießen ausgebracht. Die Anwendungsformen richten sich ganz nach den Verwendungszwecken; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron uzw., stark polare Lösungsmittel, wie z.B. Dimethylformamid, Dimethylsulfoxid, N-Methylpyrrolidon, Wasser, usw. in Betracht.

Wäßrige Anwendungsformen können aus Emulsionskonzentraten, Pasten oder netzbaren Pulvern - (Spritzpulvern), Öldispersionen durch Zusatz von Wasser bereitet werden. Zur Herstellung von Emulsionen, Pasten oder Öldisperstionen können die Substanzen als solche oder in einem Öl oder Lösungsmittel gelöst, mittels Netz-, Haft-, Dispergier-oder Emulgiermittel in Wasser homogenisiert werden. Es können aber auch aus wirksamer Substanz Netz-, Haft-, Dispergier-oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali-und Erdalkalisalze der Dibutylnaphthalinsulfonsäure, Laurylethersulfat, Fettalkoholsulfate, fettsaure Alkali-und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu-und Stäubemittel können durch Mischen oder gemeinsames Vermahlen der wirksamen Substanzen mit einem festen Trägerstoff hergestellt werden.

Granulate, z.B. Umhüllungs-, Imprägnierungs-und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Talkum, Bolus, Löß, Ton, Dolomit, Diatomeenerde, Calcium-und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz-und Nußschalenmehl, Cellulosepulver und andere feste Trägerstoffe.

Beispiele für solche Pflanzenschutzmittel-Zubereitungen sind:

I. Man vermischt 90 Gewichtsteile der Verbindung des Beispiels 1 mit 10 Gewichtsteilen N-Methylpyrrolidon und erhält eine Lösung, die zur Anwendung in Form kleinster Tropfen geeignet ist.

II. 20 Gewichtsteile der Verbindung 33 werden in eine Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung 34 werden in einer Mischung gelöst, die aus 30 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und Verrühren der Lösung in Wasser erhält man eine wäßrige Dispersion.

IV. 20 Gewichtsteile der Verbindung 36 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und Verrühren der Lösung in Wasser erhält man eine wäßrige Dispersion.

V. 20 Gewichtsteile der Verbindung 33 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der Verbindung 34 werden mit 95 Gewichtsteilen feinteiligem Kaolin innig vermischt. Man erhält auf diese Weise ein Stäubemittel, das 5 Gew.-% des Wirkstoffs enthält.

VII. 30 Gewichtsteile der Verbindung 36 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung 33 werden mit 30 Teilen Natriumsalz eines Phenolsulfonsäureharnstoff-formaldehyd-Kondensates, 2 Teilen Kieselgel und 48 Teilen Wasser innig vermischt. Man erhält eine stabile wäßrige Dispersion.

IX. 20 Teile der nach Beispiel 1 erhältlichen Verbindung werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die Wirkung der Cyclohexenonderivate der Formel I auf das Pflanzenwachstum läßt sich durch Gewächshausversuche zeigen:

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt flach eingesät. Bei Vorauflaufbehandlung werden die aufbereiteten Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha.

Nach dem Aufbringen der Mittel werden die Gefäße leicht beregnet, um Keimung und Wachstum in Gang zu bringen. Danach direkt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung betragen 0,5 und 0,25 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet. Bewertet wird nach einer Skale von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name |
|---|---|
| Alopecurus myosuroides | Ackerfuchsschwanz |
| Avena sativa | Hafer |
| Echinochloa crus galli | Hühnerhirse |

Lateinischer Name Deutscher Name

Lolium multiflorum Ital. Raygras
Medicago sativa Luzerne
Setaria italica Kolbenhirse
Setaria viridis Grüne Borstenhirse
Sinapis alba Weißer Senf
Triticum aestivum Weizen
Zea mays Mais
Avena fatua Flughafer
Digitaria sanguin. Blutfingerhirse
Setaria faberii Borstenhirse
Triticum aestivum Weizen

Die Wirkstoffe 154, 155, 153, 156 und 35 zeigen bei Vorauflaufapplikation von 3,0 kg Wirkstoff/ha eine starke herbizide Wirkung gegen Pflanzen aus der Familie der Gramineen; Sinapis alba als breitblättrige Pflanze bleibt unbeeinflußt.

Zur Bekämpfung grasartiger Vegetation eignen sich die Wirkstoffe Nr. 154 und 155 bei Nachauflaufanwendung von 0,25 kg Wirkstoff/ha. Breitblättrige Kulturen, wie am Beispiel von Luzernze gezeigt, werden dabei nicht geschädigt. Die neuen Wirkstoff haben selektive herbizide Wirkung.

Die Wirkstoffe 33 und 36 können im Nachauflaufverfahren zur Bekämpfung unerwünschter Grasarten in der Gramineenkultur Weizen eingesetzt werden. Die Nutzpflanzen erleiden keine oder allenfalls eine unwesentliche Schädigung.

Die Verbindungen Nr. 30, 31, 26, 27, 28, 65, 68, 5, 6, 7 und 8 eignen sich im Nachauflaufverfahren bei guter Verträglichkeit für Luzerne zur Bekämpfung eines breiten Spektrums unerwünschter Grasarten.

Bei guter Wirksamkeit gegen unerwünschte Grasarten im Nachauflaufverfahren schädigen die Verbindungen Nr. 71, 72, 9 und 11 die Kulturplfanze Weizen nur unwesentlich oder gar nicht.

Die Verbindungen Nr. 32, 156, 29 und 31 zeigen schon bei niedrigen Aufwandmengen bei Nachauflaufanwendung gute herbizide Wirkung gegen Grasarten ohne Luzerne zu schädigen.

Die Verbindungen 33 und 34 eignen sich zur Bekämpfung grasartiger unerwünschter Pflanzen im Nachauflaufverfahren. Sie sind darüber hinaus im Unterschied zu den Verbindungen Nr. 76 und 77 aus EP 162 224 verträglich in einer Gramineenkultur, zum Beispiel bei Weizen.

In Anbetracht des erfaßbaren Wirkungsspektrums zur Unkrautbekämpfung, der Verträglichkeit für Kulturpflanzen oder der erwünschten Beeinflussung des Wachstums derselben sowie angesichts der Vielfalt der Applikationsmethoden können die erfindungsgemäßen Verbindungen in einer großen Zahl von Kulturpflanzen eingesetzt werden. In Betracht kommen beispielsweise folgende Kulturen:

Botanischer Name Deutscher Name

Allium cepa Küchenzwiebel
Ananas comosus Ananas
Arachis hypogaea Erdnuß
Asparagus officinalis Spargel
Beta vulgaris spp. altissima Zuckerrübe
Beta vulgaris spp. rapa Futterrübe
Beta vulgaris spp. esculenta Rote Rübe
Brassica napus var. napus Raps
Brassica napus var. napobrassica Kohlrübe
Brassica napus var. rapa Weiße Rübe
Brassica rapa var. silvestris Rübsen
Camellia sinensis Teestrauch
Carthamus tinctorius Saflor -Färberdistel
Carya illinoinensis Pekannußbaum
Citrus limon Zitrone
Citrus maxima Pampelmuse
Citrus reticulata Mandarine
Citrus sinensis Apfelsine, Orange
Coffea arabica (Coffea canephora, coffea liberica) Kaffee

Cucumis melo Melone
Cucumis sativus Gurke
Cynodon dactylon Bermudagras
Daucus carota Möhre
Elaesis guineensis Ölpalme
Fragaria vesca Erdbeere
Glycine max Sojabohne
Gossypium hirsutum (Gossypium arboreum Gossypium herbaceum Gossypium vitifolium) Baumwolle
Helianthus annuus Sonnenblume
Helianthus tuberosus Topinambur
Hevea brasiliensis Parakautschukbaum
Hordeum vulgare Gerste

Botanischer Name Deutscher Name

Humulus lupulus Hopfen
Ipomoea batatas Süßkartoffeln
Juglans regia Walnußbaum
Lactua sativa Kopfsalat
Lens culinaris Linse
Linum usitatissimum Faserlein
Lycopersicon lycopersicum Tomate
Malus spp. Apfel
Manihot esculenta Maniok
Medicago sativa Luzerne
Mentha piperita Pfefferminze
Musa spp. Obst-und Mehlbanane
Nicotiana tabacum (N. rustica) Tabak
Olea europaea Ölbaum
Oryza sativa Reis
Phaseolus lunatus Mondbohne
Phaseolus mungo Erdbohne
Phaseolus vulgaris Buschbohnen
Pennisetum glaucum Perl-oder Rohrkolbenhirse
Petroselinum crispum spp. tuberosum Wurzelpetersilie
Picea abies Rotfichte
Abies alba Weißtanne
Pinus spp. Kiefer
Pisum sativum Gartenerbse
Prunus avium Süßkirsche
Prunus domestica Pflaume
Prunus dulcis Mandelbaum
Prunus persica Pfirsich
Pyrus communis Birne
Ribes sylvestre Rote Johannisbeere
Ribes uva-crispa Stachelbeere
Ricinus communis Rizinus
Saccharum officinarum Zuckerrohr
Secale cereale Roggen
Sasamum indicum Sesam
Solanum tuberosum Kartoffel
Sorghum bicolor (s. vulgare) Mohrenhirse
Spinacia oleracea Spinat
Theobroma cacao Kakaobaum

Botanischer Name Deutscher Name

Trifolium pratense Rotklee
Triticum aestivum Weizen
Vaccinium corymbosum Kulturheidelbeere
Vaccinium vitis-idaea Preißelbeere
Vicia faba Pferdebohnen
Vigna sinensis (V. unguiculata) Kuhbohne
Vitis vinifera Weinrebe

Zur Verbreiterung des Wirkungsspektrums und zur Erzielung synergistischer Effekte können die Cyclohexenonderivate der Formel I sowohl untereinander als auch mit Vertretern anderer herbizider oder wachstumsregulierender Wirkstoffgruppen gemischt und gemeinsam ausgebracht werden. Beispielsweise kommen als Mischungspartner Diazine, 4H-3,1-Benzoazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäuren und andere in Betracht.

Außerdem kann es von Nutzen sein, die Cyclohexenonderivate der Formel I bzw. sie enthaltende herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

**Ansprüche**

1. Cyclohexenonderivate der Formel

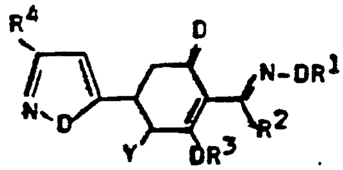

(I),

in der

$R^1$ Alkyl mit 1 bis 4 Kohlenstoffatomen, Alkenyl mit 3 bis 4 Kohlenstoffatomen, Alkinyl mit 3 bis 4 Kohlenstoffatomen, Halogenalkyl und Halogenalkenyl mit 2 bis 4 Kohlenstoffatomen und 1 bis 3 Halogenatomen, Alkoxyalkyl mit 2 bis 4 Kohlenstoffatomen, Cycloalkylmethyl mit 4 bis 7 Kohlenstoffatomen, gegebenenfalls durch $C_1$-$C_4$-Alkyl oder Halogen substituiert, Benzyl, gegebenenfalls durch $C_1$-$C_4$-Alkyl, $C_1$-$C_4$-Alkoxy, $C_1$-$C_4$-Alkylthio, Halogen, Cyan, Trifluormethyl oder Nitro substituiert, einen 5-Ring-heterocyclus, der über eine Methyleneinheit mit dem Sauerstoff verbunden ist und 1 oder 2 Heteroatome aus der Gruppe N, O, S und bis zu 2 Doppelbindungen und bis 2 Substituenten aus der Gruppe $C_1$-$C_4$-Alkyl, $C_1$-$C_4$-Alkoxy oder Halogen enthalten kann,

$R^2$ Alkyl mit 1 bis 4 Kohlenstoffatomen,

$R^3$ Wasserstoff, Alkylcarbonyl mit 1 bis 20 Kohlenstoffatomen, Alkenylcarbonyl mit 1 bis 20 Kohlenstoffatomen und einer Doppelbindung, Benzoyl, gegebenenfalls durch $C_1$-$C_8$-Alkyl substituiert, Trialkylsilyl mit 1 bis 4 Kohlenstoffatomen je Alkylrest, Alkyl-und Arylsulfonyl mit 1 bis 7 Kohlenstoffatomen, Dialkylphosphono und -thiophosphono mit 1 bis 4 Kohlenstoffatomen je Alkylrest, organisches oder anorganisches Kation,

$R^4$ $C_2$-$C_{20}$-Alkyl, Alkenyl oder Alkinyl, wobei die Kohlenstoffkette, bis zu zwei Doppel-bzw. Dreifachbindungen enthalten und durch $C_1$-$C_4$-Alkyl, $C_1$-$C_4$-Alkoxy oder $C_1$-$C_4$-Alkylthio substituiert sein kann, $C_3$-$C_6$-Cycloalkyl, gegebenenfalls durch $C_1$-$C_4$-Alkyl, $C_1$-$C_4$-Alkoxy oder $C_1$-$C_4$-Alkylthio substituiert, $C_3$-$C_8$-Alkyl mit 2 O oder S-Atomen in der Alkylkette, Methyl substituiert durch $C_1$-$C_4$-Alkoxy oder durch $C_1$-$C_4$-Alkylthio,

Y Wasserstoff, Methoxycarbonyl, Cyan, Alkyl und Halogen bedeuten.

2. Cyclohexenonderivate gemäß Anspruch 1, dadurch gekennzeichnet, daß $R^4$ Isopropyl, $R^2$ n-Propyl, $R^3$ Wasserstoff und $R^1$ Ethyl, Allyl oder Butenyl bedeutet.
der Formel

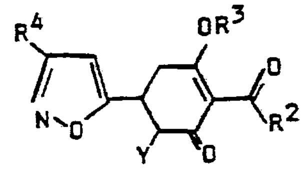

(II),

in der $R^2$, $R^4$ und Y die in Anspruch 1 genannten Bedeutungen haben,

a) mit einem Hydroxylaminderivat der Formel $R^1ONH_3Y$, in der $R^1$ die im Anspruch 1 genannte Bedeutung hat und Y ein beliebiges Anion bedeutet, in einem inertem Lösungsmittel bei einer Temperatur zwischen 0 und 80°C gegebenenfalls unter Zugabe einer Hilfsbase umsetzt

oder

b) mit einem gegebenenfalls in wäßriger Lösung vorliegenden Hydroxylamin der Formel $R^1ONH_2$, wobei $R^1$ die obige Bedeutung hat, bei Temperaturen zwischen 0 und 90°C gegebenenfalls unter Zugabe eines Lösungsmittels umsetzt.

3. Herbizid, enthaltend einen inerten Zusatzstoff und ein Cyclohexenonderivat der Formel

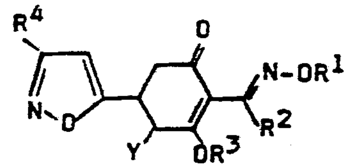

(I),

in der

$R^1$ Alkyl mit 1 bis 4 Kohlenstoffatomen, Alkenyl mit 3 bis 4 Kohlenstoffatomen, Alkinyl mit 3 bis 4 Kohlenstoffatomen, Halogenalkyl und Halogenalkenyl mit 2 bis 4 Kohlenstoffatomen und 1 bis 3 Halogenatomen, Alkoxyalkyl mit 2 bis 4 Kohlenstoffatomen, Cycloalkylmethyl mit 4 bis 7 Kohlenstoffatomen, gegebenenfalls durch $C_1$-$C_4$-Alkyl oder Halogen substituiert, Benzyl, gegebenenfalls durch $C_1$-$C_4$-Alkyl, $C_1$-$C_4$-Alkoxy, $C_1$-$C_4$-Alkylthio, Halogen, Cyan, Trifluormethyl oder Nitro substituiert, einen 5-Ring-heterocyclus, der über eine Methyleneinheit mit dem Sauerstoff verbunden ist und 1 oder 2 Heteroatome aus der Gruppe N, O, S und bis zu 2 Doppelbindungen und bis zu 2 Substituenten aus der Gruppe $C_1$-$C_4$-Alkyl, $C_1$-$C_4$-Alkoxy oder Halogen enthalten kann,

$R^2$ Alkyl mit 1 bis 4 Kohlenstoffatomen,

$R^3$ Wasserstoff, Alkylcarbonyl mit 1 bis 20 Kohlenstoffatomen, Alkenylcarbonyl mit 1 bis 20 Kohlenstoffatomen und einer Doppelbindung, Phenyl, gegebenenfalls durch $C_1$-$C_8$-Alkyl substituiert, Trialkylsilyl mit 1 bis 4 Kohlenstoffatomen je Alkylrest, Alkyl-und Arylsulfonyl mit 1 bis 7 Kohlenstoffatomen, Dialkylphosphono und -thiophosphono mit 1 bis 4 Kohlenstoffatome je Alkylrest, organisches oder anorganisches Kation,

$R^4$ $C_2$-$C_{20}$-Alkyl, Alkenyl oder Alkinyl, wobei die Kohlenstoffkette bis zu zwei Doppel-bzw. Dreifachbindungen enthalten und durch $C_1$-$C_4$-Alkyl, $C_1$-$C_4$-Alkoxy oder $C_1$-$C_4$-Alkylthio substituiert sein kann, $C_3$-$C_6$-Cycloalkyl, gegebenenfalls durch $C_1$-$C_4$-Alkyl, $C_1$-$C_4$-Alkoxy oder $C_1$-$C_4$-Alkylthio substituiert, $C_3$-$C_8$-Alkyl mit 2 O oder S-Atomen in der Alkylkette, Methyl substituiert durch $C_1$-$C_4$-Alkoxy oder durch $C_1$-$C_4$-Alkylthio,

Y Wasserstoff, Methoxycarbonyl, Cyan, Alkyl und Halogen bedeuten.

4. Verfahren zur Bekämpfung unerwünschten Pflanzenwuchses, dadurch gekennzeichnet, daß man die unerwünschten Pflanzen oder von unerwünschtem Pflanzenwachstum freizuhaltende Fläche mit einer herbizid wirksamen Menge eines Cyclohexenonderivats der Formel

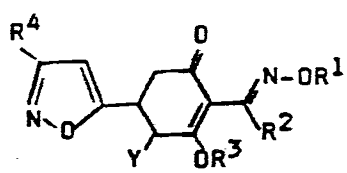

(I),

in der

$R^1$ Alkyl mit 1 bis 4 Kohlenstoffatomen, Alkenyl mit 3 bis 4 Kohlenstoffatomen, Alkinyl mit 3 bis 4 Kohlenstoffatomen, Halogenalkyl und Halogenalkenyl mit 2 bis 4 Kohlenstoffatomen und 1 bis 3 Halogenatomen, Alkoxyalkyl mit 2 bis 4 Kohlenstoffatomen, Cycloalkylmethyl mit 4 bis 7 Kohlenstoffatomen, gegebenenfalls durch $C_1$-$C_4$-Alkyl oder Halogen substituiert, Benzyl, gegebenenfalls durch $C_1$-$C_4$-Alkyl, $C_1$-$C_4$-Alkoxy, $C_1$-$C_4$-Alkylthio, Halogen, Cyan, Trifluormethyl oder Nitro substituiert, eine 5-Ring-heterocyclus, der über eine Methyleneinheit mit dem Sauerstoff verbunden ist und 1 oder 2 Heteroatome aus der Gruppe N, O, S und bis zu 2 Doppelbindungen und bis zu 2 Substituenten aus der Gruppe $C_1$-$C_4$-Alkyl, $C_1$-$C_4$-Alkoxy oder Halogen enthalten kann,

$R^2$ Alkyl mit 1 bis 4 Kohlenstoffatomen,

$R^3$ Wasserstoff, Alkylcarbonyl mit 1 bis 20 Kohlenstoffatomen, Alkenylcarbonyl mit 1 bis 20 Kohlenstoffatomen und einer Doppel bindung, Phenyl, gegebenenfalls durch $C_1$-$C_8$-Alkyl substituiert, Trialkylsilyl mit 1 bis 4 Kohlenstoffatomen je Alkylrest, Alkyl-und Arylsulfonyl mit 1 bis 7 Kohlenstoffatomen, Dialkylphosphono und -thiophosphono mit 1 bis 4 Kohlenstoffatomen je Alkylrest, organisches oder anorganisches Kation,
$R^4$ $C_2$-$C_{20}$-Alkyl, Alkenyl oder Alkinyl, wobei die Kohlenstoffkette bis zu zwei Doppel-bzw. Dreifachbindungen enthalten und durch $C_1$-$C_4$-Alkyl, $C_1$-$C_4$-Alkoxy oder $C_1$-$C_4$-Alkylthio substituiert sein kann, $C_3$-$C_6$-Cycloalkyl, gegebenenfalls durch $C_1$-$C_4$-Alkyl, $C_1$-$C_4$-Alkoxy oder $C_1$-$C_4$-Alkylthio substituiert, $C_3$-$C_8$-Alkyl mit 2 O oder S-Atomen in der Alkylkette, Methyl substituiert durch $C_1$-$C_4$-Alkoxy oder durch $C_1$-$C_4$-Alkylthio,
Y Wasserstoff, Methoxycarbonyl, Cyan, Alkyl und Halogen bedeuten, behandelt.

5. Cyclohexenonderivat gemäß Anspruch 1, dadurch gekennzeichnet, daß $R^4$ Isopropyl, $R^2$ n-Propyl, $R^3$ Wasserstoff und $R^1$ Ethyl, Allyl oder Butenyl bedeutet.

6. Herbizid gemäß Anspruch 3, dadurch gekennzeichnet, daß $R^4$ Isopropyl, $R^2$ n-Propyl, $R^3$ Wasserstoff und $R^1$ Ethyl, Allyl oder Butenyl bedeutet.

Europäisches Patentamt

# EUROPÄISCHER RECHERCHENBERICHT

Nummer der Anmeldung

EP 87103795.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| --- | --- | --- | --- |
| D,A | EP - A2 - 0 162 224 (BASF)<br>* Ansprüche 1,6,7 *<br>---- | 1,3,4,6 | C 07 D 261/08<br>A 01 N 43/80 |
| | | | **RECHERCHIERTE SACHGEBIETE (Int. Cl.4)** |
| | | | C 07 D 261/00 |

Der vorliegende Recherchenbericht wurde für alle Patentansprüche erstellt.

| Recherchenort | Abschlußdatum der Recherche | Prüfer |
| --- | --- | --- |
| WIEN | 14-05-1987 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82